# EUROPÄISCHE PATENTSCHRIFT

(11) **EP 2 875 066 B1**
(45) Veröffentlichungstag und Bekanntmachung des Hinweises auf die Patenterteilung: **28.09.2016**
(21) Anmeldenummer: 13740211.1
(22) Anmeldetag: 18.07.2013
(51) Int. Cl.: C08J 3/14, B03C 1/01, C07K 16/26, C08K 3/22, G01N 33/543, H01F 1/44, C12N 15/10, C12Q 1/24

(54) **SPHÄRISCHE, MAGNETISIERBARE POLYVINYLALKOHOL-MIKROPARTIKEL, VERFAHREN FÜR DEREN HERSTELLUNG, SOWIE DEREN VERWENDUNG**
SPHERICAL MAGNETISABLE POLYVINYL ALCOHOL MICROPARTICLES, METHOD FOR PRODUCING SAME, AND USE THEREOF
MICROPARTICULES D'ALCOOL POLYVINYLIQUE, MAGNÉTISABLES, SPHÉRIQUES, LEUR PROCÉDÉ DE FABRICATION AINSI QUE LEUR UTILISATION

(30) Priorität: 21.07.2012 DE 102012014536
(43) Veröffentlichungstag der Anmeldung: 27.05.2015
(73) Patentinhaber: PerkinElmer chemagen Technologie GmbH, 52499 Baesweiler (DE)
(72) Erfinder: OSTER, Jürgen, 52134 Herzogenrath (DE); SOMMER, Thomas, 41812 Erkelenz (DE); À BRASSARD, Lothar, 52525 Heinsberg (DE)
(74) Vertreter: Flaccus, Rolf-Dieter
(86) Internationale Anmeldenummer: PCT/EP2013/002145
(87) Internationale Veröffentlichungsnummer: WO 2014/015966

(56) Entgegenhaltungen:
- EP-A2- 1 118 676
- WO-A1-97/04862
- DE-A1- 10 103 652
- US-A1- 2003 109 618
- MULLER-SCHULTE D ET AL: "Novel magnetic microspheres on the basis of poly(vinyl alcohol) as affinity medium for quantitative detection of glycated haemoglobin", JOURNAL OF CHROMATOGRAPHY, ELSEVIER SCIENCE PUBLISHERS B.V, NL, Bd. 711, Nr. 1, 8. September 1995 (1995-09-08), Seiten 53-60, XP004038950, ISSN: 0021-9673, DOI: 10.1016/0021-9673(95)00114-3
- CHENGLI YANG ET AL: "Preparation and characterization of monodisperse superparamagnetic poly(vinyl alcohol) beads by reverse spray suspension crosslinking", JOURNAL OF POLYMER SCIENCE PART A: POLYMER CHEMISTRY, Bd. 46, Nr. 1, 1. Januar 2007 (2007-01-01) , Seiten 203-210, XP055095075, ISSN: 0887-624X, DOI: 10.1002/pola.22372
- "M-PVA Magnetic Beads", PerkinElmer chemagen , 14. April 2012 (2012-04-14), XP002718375, Gefunden im Internet: URL:http://web.archive.org/web/20120414102 058/http://www.chemagen.com/magneticbeads. html? [gefunden am 2014-01-07]
- Juk Rgen Oster ET AL: "Polyvinyl-alcohol-based magnetic beads for rapid and e$cient separation of speci"c or unspeci"c nucleic acid sequences", Journal of Magnetism and Magnetic Materials, 1. Januar 2001 (2001-01-01), Seiten 145-150, XP055095234, Gefunden im Internet: URL:http://ac.els-cdn.com/S030488530001243 9/1-s2.0-S0304885300012439-main.pdf?_tid=2 360f628-7864-11e3-898d-00000aacb362&acdnat =1389185903_07872c9cdff5be89df7dcc711e8f29 80 [gefunden am 2014-01-08]

## Beschreibung

Die vorliegende Erfindung betrifft sphärische, magnetisierbare Polyvinylalkohol-Mikropartikel, die insbesondere zur Verwendung bei diagnostischen Nachweisverfahren oder zur Isolierung und Reinigung von Biomolekülen geeignet sind. Die Erfindung bezieht sich ferner auf Herstellungsverfahren, mit denen diese Mikropartikel auch in größerem Maßstab produziert werden können.

Magnetpartikel der oben erwähnten Art werden bei einer Vielzahl von diagnostischen Verfahren oder in der biomedizinischen oder molekularbiologischen Forschung eingesetzt. Im allgemeinen handelt es sich dabei um sphärische Partikel, die ein superparamagnetisches Material in kolloidaler Form enthalten, das in eine Polymermatrix eingebettet oder von einer Polymerhülle ummantelt ist. Typischerweise handelt es sich bei diesen Magnetpartikeln um Mikropartikel mit einer Größe im Bereich von 1 bis 20 *µ*m.

Aufgrund ihrer Eigenschaft, Zielsubstanzen wie z. B. Biomoleküle oder Zellen selektiv und ggf. reversibel binden zu können, eignen sich magnetisierbare Mikropartikel insbesondere für die Verwendung in automatisierten Verfahren. Da die magnetisierbaren Mikropartikel durch Anlegen eines Magnetfelds vorübergehend immobilisiert werden können, sind für die Abtrennung aus flüssigen Medien keine Zentrifugationsschritte erforderlich. Dies erleichtert die automatisierte Durchführung auch bei mehrstufigen Verfahren und bringt eine erhebliche Zeitersparnis mit sich.

Magnetisierbare Mikropartikel auf Polymerbasis, ihre Herstellung und Verwendung sind bereits im Stand der Technik beschrieben worden, beispielsweise in WO 97/04862 A1 und in den dort zitierten Druckschriften.

WO 97/04862 A1 offenbart perl- oder kugelförmige Partikel aus Polyvinylalkohol, die als zur Fraktionierung von Zellen, Nukleinsäuren, Proteinen, Viren oder Bakterien sowie zur Verwendung in Immunoassays, für die DNA-Sequenzierung oder DNA-Synthese geeignet beschrieben werden.

Die in WO 97/04862 A1 beschriebenen Partikel weisen eine Polymermatrix aus Polyvinylalkohol auf, in welche magnetische Kolloide mit Partikelgrößen von 10-200 nm eingekapselt sind. Die perl- oder kugelförmigen Polymerpartikel haben eine Partikelgröße im Bereich von 1-10 *µ*m, vorzugsweise 1-4 *µ*m.

Die Herstellung der in WO 97/04862 A1 beschriebenen magnetisierbaren Polymerpartikel erfolgt in der Weise, dass eine wässrige Polyvinylalkohol-Lösung, in der ein magnetisches Kolloid dispergiert ist, bei Raumtemperatur in einer mit der Polymerphase nicht mischbaren organischen Phase (z. B. Pflanzenöl), die mindestens zwei Emulgatoren enthält, unter Rühren suspendiert wird. Während dieses Suspensionsvorgangs erfolgt eine Vernetzung des Polyvinylalkohols durch Zugabe eines wasserlöslichen, mit Hydroxylgruppen reagierenden Vernetzers, beispielsweise Glutaraldehyd. Die Polyvinylalkohol-Partikel können anschließend zur spezifischen Bindung von Biomolekülen modifiziert werden, beispielsweise durch Aufpfropfen von Spacermolekülen, welche zur Bindung von Biomolekülen dienen können.

Es hat sich jedoch gezeigt, dass das in WO 97/04862 A1 beschriebene Herstellungsverfahren und die damit erhältlichen Partikel in verschiedener Hinsicht nachteilig sind.

Problematisch ist vor allem, dass die nach diesem bekannten Verfahren hergestellten magnetisierbaren Polymerpartikel eine zu breite Partikelgrößenverteilung aufweisen und die Partikelgrößenverteilung von Charge zu Charge schwanken kann, d. h. die Chargen-Reproduzierbarkeit ist unbefriedigend. Diese schwankenden Werte führen bei der Anwendung der Partikel in Separationsverfahren zu ungleichmäßigen Ausbeuten, so dass diese Partikel für viele Anwendungen nicht brauchbar sind.

Hinzu kommt, dass die nach dem in WO 97/04862 A1 beschriebenen Verfahren hergestellten magnetisierbaren Polymerpartikel hohe Anteile von Partikeln mit einer Größe von weniger als 0,5 *µ*m und mit einer Größe von mehr als 3 *µ*m aufweisen.

Partikel mit einer Größe von weniger als 0,5 *µ*m weisen keine ausreichende Abtrennungsgeschwindigkeit bzw. Mobilität in einem äußeren Magnetfeld auf, so dass sie bei den üblicherweise verwendeten Magnetfeldstärken nur sehr langsam separiert werden, was sich nachteilig auf die Gesamtverarbeitungszeit auswirkt. Zudem besteht das Risiko, dass diese nur langsam bzw. nicht abtrennbaren Partikel als Verunreinigungen weiter verschleppt werden und nachfolgende Reaktionen oder Messungen, z. B. UV-Messungen oder PCR-Reaktionen, stören und verfälschen.

Partikel mit einer Größe von mehr als 3 *µ*m haben den Nachteil, dass sie relativ schnell im Schwerefeld sedimentieren, wodurch die Bindungskapazität für Biomoleküle deutlich eingeschränkt wird. Dadurch kann es notwendig werden, der Sedimentation durch entsprechende Maßnahmen (Re-Dispergierung) entgegenzuwirken. Außerdem weisen die magnetisierbaren Polymerpartikel mit zunehmender Partikelgröße eine geringere Gesamtoberfläche auf, bezogen auf die Gesamtmasse bzw. das Suspensionsvolumen. Dies hat wiederum eine verringerte Ausbeute an abzutrennenden Substanzen (Biomoleküle, Zellen) zur Folge. Insgesamt wird sowohl durch die Anwesenheit zu kleiner Partikel (< 0,5 *µ*m) als auch durch die Anwesenheit zu großer Partikel (> 3 *µ*m) der magnetische Separationsprozess, beispielsweise bei der automatisierten Nukleinsäureaufreinigung, erschwert.

Des weiteren weisen die mit den in WO 97/04862 A1 beschriebenen Verfahren hergestellten magnetisierbaren Polyvinylalkoholpartikel nur einen relativ niedrigen Gehalt an magnetisierbarem Material (Magnetit/Eisenoxid) auf, nämlich im Bereich von ca. 7 bis 24 Gew.-%. Dies führt zu ungenügenden oder ungünstigen Separationseigenschaften der Partikel im Magnetfeld.

Aufgrund der vorstehend beschriebenen Nachteile hinsichtlich der Partikelgrößen, der Partikelgrößenverteilung und des Magnetitgehalts sind die nach dem in WO 97/04862 A1 beschriebenen Verfahren hergestellten magnetisierbaren Polymerpartikel nur eingeschränkt brauchbar und insbesondere für die Verwendung in automatisierten Separations- und Analyseverfahren wenig geeignet.

Als besonders nachteilig hat sich herausgestellt, dass die im Stand der Technik bekannten magnetisierbaren Polymerpartikel bei der Verwendung in PCR-Verfahren inhibitorisch wirken, wodurch die Nachweisempfindlichkeit verringert und die Genauigkeit bzw. Zuverlässigkeit der Messungen beeinträchtigt wird. Da PCR-Techniken in der molekularbiologischen Forschung und in der medizinischen Diagnostik weit verbreitet sind und die Verwendung von magnetisierbaren Mikropartikeln im Hinblick auf die Automatisierung dieser Methoden an Bedeutung zunimmt, ist die erwähnte inhibierende Wirkung der im Stand der Technik bekannten Partikel höchst unbefriedigend.

Des weiteren hat sich gezeigt, dass das in WO 97/04862 A1 beschriebene Herstellungsverfahren nicht für die Produktion von magnetisierbaren Polyvinylalkohol-Partikeln in größerem Maßstab geeignet ist. Bei diesem bekannten Verfahren ist das Volumen der Reaktionsansätze auf maximal ca. 5 1 beschränkt, und die Ausbeute an magnetisierbaren Polymerpartikeln ist zu niedrig. Deshalb ist es mit diesem bekannten Herstellungsverfahren nicht möglich, magnetisierbare Polyvinylalkohol-Partikel in größeren Mengen und mit den geforderten Qualitätseigenschaften (insbesondere mit einer engen Partikelgrößenverteilung) kostengünstig zu produzieren.

In Anbetracht der vorstehend genannten Nachteile und Anforderungen bestand die der vorliegenden Erfindung zugrunde liegende Aufgabe darin, magnetisierbare PolyvinylalkoholPartikel der oben beschriebenen Art bereitzustellen, die sich vor allem durch eine enge Partikelgrößenverteilung im Bereich von 0,5-3 *µ*m auszeichnen und die bei den bestimmungsgemäßen Anwendungen, insbesondere beim Einsatz in automatisierten Anwendungsverfahren, eine hohe Verarbeitungsgeschwindigkeit (d. h. einen hohen Durchsatz), eine verbesserte Effizienz bei der Abtrennung der Zielsubstanzen (insbesondere hinsichtlich der Produktreinheit) oder/und erhöhte Ausbeuten ermöglichen, und die bei PCR-Anwendungen keine inhibierende Wirkung oder nur eine geringfügige inhibierende Wirkung verursachen.

Ferner lag der vorliegenden Erfindung die Aufgabe zugrunde, Herstellungsverfahren bereitzustellen, mittels welchen die erfindungsgemäßen magnetisierbaren Mikropartikel produziert werden können, insbesondere chargen-konsistent produziert werden können. Des weiteren lag der vorliegenden Erfindung die Aufgabe zugrunde, Herstellungsverfahren bereitzustellen, mittels welcher die magnetisierbaren Mikropartikel in größerem Maßstab und mit der angegebenen engen Partikelgrößenverteilung produziert werden können, d. h. in Mengen von 200 g oder mehr, vorzugsweise von 500 g oder mehr, insbesondere von 1 kg oder mehr, jeweils pro Reaktionsansatz.

Die vorstehend genannten Aufgaben werden durch die in den unabhängigen Patentansprüchen beanspruchten Herstellungsverfahren und sphärischen, magnetisierbaren Polyvinylalkohol-Mikropartikel gelöst, sowie durch die in den abhängigen Ansprüchen definierten, besonderen oder bevorzugten Ausführungsformen, und durch die weiteren Ausführungsformen, die sich aus der nachfolgenden Beschreibung ergeben. Mit den erfindungsgemäßen Verfahrensmerkmalen, insbesondere in ihrer Gesamtheit, lassen sich sphärische, magnetisierbare Polyvinylalkohol-Mikropartikel mit den erwähnen verbesserten Partikeleigenschaften erhalten, wie nachfolgend erläutert.

Das erfindungsgemäße Verfahren, welches die Herstellung von sphärischen, magnetisierbaren Polyvinylalkohol-Mikropartikeln mit einer engen Partikelgrößenverteilung im Bereich von 0,5 bis 3 *µ*m und/oder mit weiteren vorstehend genannten vorteilhaften Eigenschaften im Produktionsmaßetab (z. B. 200 g pro Ansatz oder mehr) ermöglicht, umfasst zumindest die folgenden Schritte:
(1) Dispergieren eines nanopartikulären magnetisierbaren Materials, vorzugsweise Magnetit, in einer wässrigen Phase, die Polyvinylalkohol (PVA) in gelöster Form enthält (wässrige Polyvinylalkohol-Lösung);
(2) Hinzufügen dieser wässrigen Phase zu einer organischen Phase, welche mit der wässrigen Phase nicht mischbar ist und mindestens einen Emulgator enthält, und Erzeugen einer Emulsion durch Rühren bei einer Temperatur von 40 °C oder höher;
(3) Hinzufügen mindestens einer zur Vernetzung von Polyvinylalkohol geeigneten, wasserlöslichen Vernetzer-Substanz unter fortgesetztem Rühren.

Ohne sich auf eine bestimmte Theorie festlegen zu wollen, wird angenommen, dass die Bildung der sphärischen, magnetisierbaren Polyvinylalkoholpartikel in der Weise erfolgt, dass sich während des Dispergierens (Schritt (1)) auf den einzelnen Partikeln des nanopartikulären magnetisierbaren Materials (z. B. Magnetit) dünne PVA-Schichten ausbilden, wodurch die Bildung von Partikel-Agglomeraten verhindert wird. In der gemäß Schritt (2) erzeugten Emulsion bildet die wässrige Phase, welche PVA und die mit PVA beschichteten, magnetisierbaren Nanopartikel enthält, zusammen mit der organischen Phase und dem Emulgator eine Emulsion mit sphärischen Micellen aus. Durch die Vernetzung (Schritt (3)) werden die Nanopartikel mittels kovalenter Verknüpfung vieler PVA-Ketten an der Partikeloberfläche stabilisiert.

Insbesondere aufgrund der höheren Emulgierungstemperatur (Schritt (2), 25 °C oder höher) wird die Bildung von Partikeln mit zu geringer Partikelgröße (insbesondere < 0,5 *µ*m) unterdrückt und das Größenmaximum zu größeren Partikeln hin verschoben; die dabei resultierenden Partikel weisen ein optimales Verhältnis von Magnetisierbarkeit (und Abtrennbarkeit) zu Oberflächenkapazität und Sedimentationsverhalten auf. Ferner wird durch die erfindungsgemäßen Verfahren eine effizientere Einkapselung des magnetisierbaren nanopartikulären Materials in der Polyvinylalkohol-Schicht erreicht; hieraus resultiert die erwähnte Verminderung bzw. Abwesenheit von inhibitorischen Wirkungen.

Die mit den erfindungsgemäßen Verfahren erhältlichen Partikel sind aufgrund der vorstehend beschriebenen Herstellungsmethode im wesentlichen sphärisch, d. h. kugel- oder perlförmig, wie im Lichtmikroskop erkennbar.

Die Bezeichnung "magnetisierbar" bedeutet, dass die Partikel in der Weise magnetisierbar sind, dass sie unter Einwirkung eines externen magnetischen Feldes magnetisch angezogen und beispielsweise aus einem flüssigen Medium abgetrennt werden können. Beim Abschalten des magnetischen Feldes wird die Magnetisierung der Partikel wieder aufgehoben, d. h. die Partikel sollten vorzugsweise eine Remanenz von nahezu Null oder von Null aufweisen.

Die Bezeichnung "Polyvinylalkohol-Mikropartikel" bedeutet, dass die erfindungsgemäßen magnetisierbaren Mikropartikel einen Gehalt an Polyvinylalkohol aufweisen. Dieses Polymer bildet, gegebenenfalls in Kombination mit weiteren Substanzen, eine Matrix, in welcher das nanopartikuläre magnetisierbare Material eingebettet oder eingeschlossen ist. Gemäß einer bevorzugten Ausführungsform besteht die polymere Matrix der magnetisierbaren Mikropartikel ausschließlich aus Polyvinylalkohol.

Die mit den erfindungsgemäßen Verfahren erhältlichen magnetisierbaren Polyvinylalkohol-Mikropartikel weisen eine enge Partikelgrößenverteilung im Bereich von 0,5 bis 3 *µ*m, vorzugsweise von 0,5 bis 1 *µ*m, auf. Gemäß einer weiteren bevorzugten Ausführungsform weisen die PolyvinylalkoholMikropartikel eine Partikelgrößenverteilung im Bereich von 1,25 bis 2,25 *µ*m auf.

Die Angabe "enge Partikelgrößenverteilung" bedeutet insbesondere, dass mindestens 75 %, vorzugsweise mindestens 90 %, besonders bevorzugt mindestens 95 % der Partikel, eine Partikelgröße aufweisen, die innerhalb des angegebenen Größenbereichs von 0,5 bis 3 *µ*m bzw. von 0,5 bis 1 *µ*m liegt.

Die Partikelverteilung ist vorzugsweise monomodal, d. h. sie weist innerhalb des Bereichs von 0,5 bis 3 *µ*m bzw. innerhalb des Bereichs von 0,5 bis 1 *µ*m ein einziges Maximum auf.

Als nanopartikuläres magnetisierbares Material, welches in der wässrigen Polyvinylalkohol-Lösung dispergiert wird, können insbesondere ferromagnetische oder superparamagnetische Kolloid-Partikel verwendet werden, wobei nanokristalliner Magnetit besonders bevorzugt wird.

Vorzugsweise weist das nanopartikuläre magnetisierbare Material eine Partikelgröße im Bereich von 5 bis 250 nm, insbesondere 5 bis 100 nm, besonders bevorzugt 5 bis 50 nm auf.

Nach einer bevorzugten Ausführungsform der Erfindung wird das verwendete magnetisierbare nanopartikuläre Material, vorzugsweise Magnetit-Nanopartikel, durch ein Verfahren hergestellt oder vorbehandelt, welches sich durch folgende Merkmale auszeichnet :
- Suspendieren von magnetisierbaren Nanopartikeln in vollentsalztem Wasser mit einem Leitwert von weniger als 500 *µ*S/cm, vorzugsweise weniger als 100 *µ*S/cm, besonders bevorzugt weniger als 5 *µ*S/cm;
- Behandeln der wässrigen Nanopartikel-Suspension mittels eines Ultraschall-Homogenisators.

Die anfänglich eingesetzten magnetisierbaren Nanopartikel können mittels bekannter Methoden erzeugt werden (siehe z. B. Sipos P.: "Manufacturing of Size Controlled Magnetite Nanoparticles Potentially Suitable for the Preparation of Aqueous Magnetic fluids"; Romanian reports in physics 2006; 58(3): 229-233.; sowie: YE XR et al.: "Room temperature solvent-free synthesis of monodisperse magnetite nanocrystals", J. Nanosci. Nanotechnol. 2006, Vol. 6, No. 3, pp. 852-856).
Vorzugsweise erfolgt die Herstellung der Nanopartikel wie auch deren weitere Behandlung ohne Zusatz von oberflächenaktiven Substanzen.

Durch die erwähnte Ultraschallbehandlung der Nanopartikelsuspension kann eine weitgehende Zerstörung von eventuell vorhandenen Partikelaggregaten erreicht werden. Die Ultraschallbehandlung wird vorzugsweise im Durchflussverfahren durchgeführt.

Die Ultraschall-Leistung beträgt dabei vorzugsweise mindestens 1000 W. Die Dauer der Beschallung beträgt vorzugsweise mindestens 10 min, besonders bevorzugt mindestens 0,5 h, insbesondere mindestens 1 h.

Geeignete Ultraschall-Homogenisatoren zur Durchführung der erwähnten Ultraschallbehandlung sind dem Fachmann bekannt bzw. im Handel erhältlich (z. B. Dr. Hielscher Sonopuls 2000 W; Hielscher Ultrasonics GmbH, D-14513 Teltow).

Des weiteren hat es sich als vorteilhaft erwiesen, wenn die Nanopartikel-Suspension vor ihrer weiterer Verwendung einem Zentrifugationsschritt unterzogen wird, bei welchem Partikel mit einer Größe > 250 nm, vorzugsweise mit einer Größe von > 100 nm, entfernt werden. Die Zentrifugation wird vorzugsweise bei 1000-3000 x g durchgeführt.

Es wurde gefunden, dass sich die mittels der vorstehend beschriebenen Methoden vorbehandelten magnetisierbaren Nanopartikel in besonderer Weise für die Herstellung der erfindungsgemäßen, magnetisierbaren Polyvinylalkohol-Mikropartikel eignen, welche die oben beschriebenen vorteilhaften Eigenschaften (insbesondere eine enge Partikelgrößenverteilung im angegebenen Größenbereich) aufweisen.

Durch die Verwendung von nanopartikulären magnetisierbarem Material, insbesondere Magnetit, mit Korngrößen von maximal 250 nm, vorzugsweise von maximal 100 nm, lassen sich sphärische, magnetisierbare Polyvinylalkohol-Mikropartikel herstellen, die einen relativ hohen Anteil an magnetisierbarem Material, insbesondere Magnetit, aufweisen.

Vorzugsweise liegt dieser Anteil bei mindestens 50 Gew.-%. Gemäß einer besonders bevorzugten Auaführungsform weisen die erfindungsgemäßen Partikel einen Magnetitgehalt von 50 bis 60 Gew.-% auf (Mittelwert: ca. 55 Gew.-%).

Bei dem zur Herstellung der wässrigen Phase verwendeten Polyvinylalkohol handelt es sich vorzugsweise um Polyvinylalkohol mit einer mittleren Molmasse im Bereich von 50.000 bis 300.000 und einen Hydrolysegrad im Bereich von 70 bis 99,9 Mol-%, vorzugsweise 80 bis 95 Mol-%. Beispielsweise kommen für diesen Zweck die unter der Bezeichnung "Mowiol" erhältlichen Polyvinylalkohol-Typen in Betracht (Kuraray Europe GmbH, Frankfurt a. M.).

Die Polyvinylalkohol-Konzentration in der wässrigen Phase beträgt vorzugsweise höchstens 2 Gew.-%, insbesondere 0,1 bis 2 Gew.-%, besonders bevorzugt 0,5 bis 1,5 Gew.-%.

Es hat sich gezeigt, dass die Beschränkung der Polyvinylalkohol-Roazeatration in der wässrigen Phase auf höchstens 2,0 Gew.-%, insbesondere auf 1,5 Gew.-%, wesentlich dazu beiträgt, um die Bildung von Partikeln mit zu großem Durchmesser (insbesondere von mehr als 3 *µ*m) vermeiden, die sich aufgrund ihrer Sedimentationseigenschaften ungünstig bei der späteren Verwendung der Partikel, insbesondere bei der automatisierten Nukleinsäure-Aufreinigung, auswirken würden. Des weiteren trägt die Beschränkung der Polyvinylalkohol-Ronzentration auf maximal 2,0, insbesondere 1,5 Gew.-%, dazu bei, dass magnetisierbare Polymerpartikel mit einem hohen Anteil an magnetisierbarem Material (z. B. Magnetit) erhalten werden können, insbesondere mit einem Anteil von 50 Gew.-% oder mehr, besonders bevorzugt 50 bis 60 Gew.-%.

Das magnetisierbare Material, vorzugsweise Magnetit, wird der wässrigen Phase vorzugsweise in einem Anteil von 0,5 bis 7,5 Gew.-% zugesetzt, besonders bevorzugt 1 bis 5 Gew.-%.

Der Dispergiervorgang im ersten Schritt des Verfahrens kann mittels bekannter Methoden durchgeführt werden, üblicherweise unter Verwendung eines Dispergiergeräts (z. B. Dispergiergerät ULTRA-TURRAX®) oder eines Propeller-Rührers. Sofern erforderlich, kann der Dispergiervorgang unter Erwärmen durchgeführt werden, um die möglichst vollständige Lösung des Polyvinylalkohols sicherzustellen.

In einer bevorzugten Ausführungsform des Verfahrens wird die Polyvinylalkohol und magnetisierbares Material enthaltende wässrige Phase vor dem Hinzufügen zur organischen Phase mittels eines Ultraschall-Homogenisators homogenisiert. Dadurch kann das magnetisierbare Material, sofern gewünscht oder erforderlich, auf eine Partikelgröße von weniger als 250 nm, vorzugsweise von weniger als 100 nm (hydrodynamischer Durchmesser) zerkleinert werden. Dieser zusätzliche Homogenisierungs-Schritt hat ferner den Vorteil, dass eventuell vorhandene Partikel-Aggregate oder Verklumpungen zerkleinert werden. Geeignete Ultraschall-Homogenisatoren sind dem Fachmann bekannt und im Handel erhältlich (z. B. "Labsonic Pⁿ**,** Fa. Sartorius AG, Göttingen; "Dr. Hielscher Sonopuls 2000 W";).

Auf den Zusatz von Emulgatoren zur wässrigen Polymerphase kann aufgrund der besonderen Merkmale der erfindungsgemäßen Herstellungsverfahren verzichtet werden.

In einem zweiten oder weiteren Schritt des erfindungsgemäßen Herstellungsverfahrens wird die wässrige Phase zu einer organischen Phase hinzugefügt, welche mit der wässrigen Phase nicht mischbar ist und mindestens einen Emulgator enthält. Sodann wird unter Rühren bei einer Temperatur von 40 °C oder höher eine Emulsion erzeugt. Vorzugsweise liegt die Temperatur bei mindestens 50 °C, insbesondere bei 55 bis 65 °C, am meisten bevorzugt bei 60 °C**.** Überraschenderweise hat sich gezeigt, dass die Bildung sehr kleiner Partikel (weniger als 0,5 *µ*m) reduziert oder verhindert werden kann, wenn die Herstellung der Partikel bei einer erhöhten Temperatur, wie angegeben, erfolgt, wobei die angegebenen höheren Temperaturen (≥ 30 °C, ≥ 40 °C, ≥ 50 °C, ≥ 60 °C) sich hierbei besonders günstig auswirken.

Der Emulsionsvorgang ist - abhängig von der Art des verwendeten Dispergier- oder Rührgeräts, der Größe des Emulsionsvolumens etc. - im allgemeinen nach 10 s bis 15 min abgeschlossen.

Das Emulgieren kann auf dem Fachmann bekannte Weise mittels eines üblichen Dispergier- oder Rührgeräts erfolgen, beispielsweise bei Rührgeschwindigkeiten im Bereich von 500 bis 5000 U/min.

Für die Herstellung der Emulsion hat sich gemäß vorliegender Erfindung die Verwendung eines nach dem Rotor-Stator-Prinzip arbeitenden Dispergierrührers als besonders vorteilhaft erwiesen. Die verwendete Rotordrehzahl liegt typischerweise im Bereich von 500 bis 4000 U/min. Üblicherweise taucht der Mischkopf des Dispergierrührers dabei in die zu emulgierende Flüssigkeit ein, die sich in einem geschlossenen Behälter befindet. Das Fassungsvolumen des Behälters kann bis zu 100 1, beispielsweise 10 bis 100 1, oder einige 100 1 betragen, beispielsweise 100 bis 500 1.

Der Dispergier- und Emulsionsvorgang erfolgt unter Verwendung des erwähnten Dispergierrührers im wesentlichen ohne Lufteintrag. Vorzugsweise ist der Dispergierrührer mit einem Mischkopf ausgestattet, dessen Stator (der den Rotor umgibt) mit einer Vielzahl von vertikalen Schlitzen versehen ist. Während der Rotation entsteht eine vertikale, nach unten gerichtete, sowie eine horizontale Flüssigkeitsströmung; letztere bewirkt, dass die zu emulgierende Flüssigkeit durch die Schlitze des Mischkopfes geführt wird, was wegen der dabei auftretenden hohen Scherkräfte und der im Mischkopf entstehenden Turbulenzen zu einer sehr effizienten Durchmischung und Homogenisierung führt. Insbesondere wird dadurch eine schnelle Verteilung des Vernetzers in der Suspension begünstigt.

Dispergierrührer der vorstehend beschriebenen Art sind dem Fachmann bekannt; beispielsweise kann ein Dispergierrührer der Fa. ystral GmbH verwendet werden ("ystral Dispermix", Fa. ystral GmbH, D-79282 Ballrechten-Dottingen).

Bei Verwendung eines Dispergierrührers, wie vorstehend beschrieben, wird eine besonders effiziente Desagglomerierung und Suspendierung erzielt; zudem können aufgrund der hohen Umwälzleistung des Dispergierrührers eine punktuelle übers hitzungen verhindert und eine schnelle Verteilung des Vernetzers bewirkt werden. Damit wird das unerwünschte "Zusammenlaufen" von Micellen unterdrückt bzw. verhindert, d. h. die Entstehung von "Mehrfach-Beads" oder Vereinigung zweier oder mehrerer Micellen wird gehemmt.

Es wurde gefunden, dass die Verwendung eines Dispergierrührers der vorstehend beschriebenen Art insbesondere dann vorteilhaft ist, wenn große Emulsionsvolumina (10 1 oder mehr) verarbeitet werden sollen, bei möglichst gleichbleibender Chargen-Konsistenz.

Die vorstehend beschriebene Dispergiermethode ist nicht nur besonders vorteilhaft im Hinblick auf die Verarbeitung größerer Ansatzvolumina, sondern auch im Hinblick auf die Steigerung der Ausbeute an magnetisierbaren Polyvinylalkohol-Mikropartikeln. Beispielsweise beträgt die Ausbeute an magnetisierbaren, sphärischen Polyvinylalkohol-Partikeln bei einem Ansatzvolumen (Emulsionsvolumen) von 100 1 mindestens 200 g.

Als nicht wassermischbare organische Phase kommen insbesondere folgende Flüssigkeiten in Betracht: Pflanzenöle (z. B. Raps- oder Sonnenblumenöl), Mineralöle, synthetische Öle, Silikonöle und Paraffinöle; sowie Mischungen der vorgenannten Öle.

Die organische, nicht wassermischbare Phase wird im allgemeinen im Volumenüberschuss relativ zur wässrigen Phase eingesetzt. Der Volumenanteil der organischen, nicht mit Wasser mischbaren Phase entspricht vorzugsweise dem 3- bis 50-fachen, insbesondere dem 3- bis 25-fachen Volumen, besonders bevorzugt dem 3- bis 15-fachen Volumen der wässrigen Phase.

Der bei der Herstellung der Emulsion verwendete Emulgator wird vorzugsweise aus der folgenden Gruppe von Emulgatoren ausgewählt: Propylenoxid-Ethylenoxid-Blockcopolymere (Poloxamere, z. B. Syaperonic^{®}**,** Tetronic^{®}, Pluronic^{®}), Polysorbate (Verbindungen, die durch Veretherung von Sorbitanfettsäureestern mit Polyethylenglycol gebildet sind; z. B. Tween^{®}, bevorzugt Tween^{®} 80), Sorbitan-Fettsäureester (z. B. Sorbitanlaurat, Sorbitanstearat, Sorbitanoleat, Sorbitansesquioleat; z. B. Arlacel^{®}, Span^{®}, Dehymuls^{®}), polyethoxylierte Fettsäuren (ethoxylierte Fettsäure-Ester, Fettsäure-Ethanolamide, Alkylamin-Ethoxylate (z. B. Lauryl-, Oleyl oder Stearylamin-PEG-Ether, Triethanolamin-PEG-Ether), Additionsprodukte von Ethylenoxid oder/und Propylenoxid an Fettalkohole mit 8 bis 18 C-Atomen (z. B. Brij^{®}, Eumulgin^{®}**,** Polidocanol), polyoxyethyliertes hydriertes Ricinusöl (z. B. DEHYMULS^{®} HRE 7), Blockcopolymere aus Ricinusöl-Derivaten, Polyoxyethylen-Polyoxypropylen-Ethylendiamin-Blockcopolymere, Polyoxyethylen-Blockcopolymere aus einer Polyhydroxyfettsäure und Polyethylenoxid (z. B. Hypermer^{®} A70), Alkylphenolethoxylate (insbesondere Octylphenol oder Nonylphenol mit 2-100 Ethylenoxyd-Einheiten; z. B. Triton^{®}, Triton^{®}X-100), Alkylphenolpropoxylate, Pentaerythrit-Fettsäureester (z.B. Pentraerythrityl-Monolaurat), Mischester aus Pentaerythrit-Fettsäureester und Citronensäure-Fettalkoholester (z. B. Dehymuls^{®} FCE), Polyäthylenglykole, Alkylbenzolsulfonsäuren und -sulfonate, Partialester von Polyglycerin, Zuckeralkoholen oder Alkylglycosiden mit gesättigten und/oder ungesättigten, linearen oder verzweigten Fettsäuren mit 8 bis 22 Kohlenstoffatomen, Polyhydroxyfettsäure-Polyethylenglykol-Blockcopolymere, und Phosphoglyceride wie Phosphatidylcholin oder Phosphatidylethanolamin. Des weiteren können auch Mischungen bzw. Kombinationen von zwei oder mehreren Emulgatoren verwendet werden.

Eine bevorzugte Mischung von Emulgatoren enthält folgende Hauptbestandteile: (1) Tween^{®} 80 (Polyoxyethylen(20)-sorbitan-monooleat) und/oder (2) Arlacel^{®} 83 (Sorbitansesquioleat); (3) DEHYMULS^{®} HRE 7 (ethoxyliertes(PEG-7), hydriertes Rizinusöl und (4) Hypermer^{®} A70 (Polyoxyethylen-Blockcopolymere aus einer Polyhydroxyfettsäure und Polyethylenoxid).

Bevorzugt werden Mischungen von Emulgatoren verwendet, die mindestens einen lipophilen Emulgator in Kombination mit mindestens einem hydrophilen oder amphiphilen Emulgator enthalten. Lipophile Emulgatoren sind im allgemeinen nichtionische Tenside mit HLB-Werten im Bereich von 3 bis 8; diese werden auch als W/O-Emulgatoren bezeichnet. Hydrophile oder amphiphile Emulgatoren haben typischerweise HLB-Werte im Bereich von 8 bis 18 und werden auch als O/W-Emulgatoren bezeichnet.

Zur Gruppe der lipophilen Emulgatoren gehören beispielsweise folgende: Sorbitan-Fettsäureester (z. B. Span^{®} 40), Lecithin, PEG-PPG-PEG-Blockcopolymere (z. B. Pluronic^{®} L31, L61, L81), PEG-Oleylether (z. B. Hrij^{®} 93).

Zur Gruppe der hydrophilen oder amphiphilen Emulgatoren gehören beispielsweise folgende: PEG-PPG-PEG-Blockcopolymere (z. B. Pluronic^{®} L-64, Pluronic^{®} 10R5), PEG-Hexadecylether (z. B. Brij^{®} C10), PEG-Octadecylether (z. B. Hrij^{®} S10), Polyoxyethylen-Nonylphenylether, Polyoxyethylen-tridecylether, Polyoxyethylen-Sorbitanmonostearat (z. B. Tween^{®} 60), Polyoxyethylen-Sorbitanmonolaurat (z. B. Tween^{®} 20), Polyoxyethylen-stearylether (z. B. Brij^{®} S100), polyethoxyliertes hydriertes Ricinusöl.

Der Emulgator bzw. die Mischung von zwei oder mehr Emulgatoren kann der nicht wassermischbaren Phase (Ölphase) zugesetzt werden bzw. darin gelöst werden, bevor diese Phase mit der wässrigen Phase zwecks Emulgierung vereinigt wird.

Der Anteil des Emulgators bzw. der Emulgatoren beträgt vorzugsweise 0,1 bis 10 Gew.-%, vorzugsweise 1 bis 7,5 Gew.-%, jeweils bezogen auf die organische Phase.

Während des Emulgierungsschrittes oder im Anschluss daran erfolgt der Zusatz mindestens einer zur Vernetzung von Polyvinylalkohol geeigneten, wasserlöslichen Vernetzer-Substanz, wodurch die das magnetisierbare Material einschließenden, sphärischen Polyvinylalkohol-Partikel durch kovalente Vernetzung stabilisiert werden.

Die Zugabe der Vernetzer-Substanz(en) erfolgt vorzugsweise während des Emulgierens, d. h. die beiden Phasen werden zunächst in Abwesenheit des Vernetzers durch Rühren dispergiert bzw. emulgiert, woraufhin der Vernetzer hinzugefügt und der Rührvorgang fortgesetzt wird, bis die Vernetzungsreaktion abgeschlossen ist (im allgemeinen ca. 10 s bis 5 min nach Zugabe des Vernetzers). Vorzugsweise wird die Vernetzung ebenfalls bei einer erhöhten Temperatur, wie oben für den Emulgierungsschritt angegeben, durchgeführt. Die beim Emulgieren verwendete erhöhte Temperatur (> 25 °C) kann auch während der Vernetzungsreaktion beibehalten werden.

Als Vernetzer-Substanzen werden vorzugsweise bifunktionale Aldehyde, insbesondere Glutaraldehyd, sowie Säurechloride oder Divinylsulfon verwendet, wobei Glutaraldehyd besonders bevorzugt wird. Hingegen hat sich die Verwendung von Diaminen als Vernetzer (z. B. Hexamethylendiamin) als ungünstig erwiesen, weil die freien Aminogruppen unerwünschte Nebenreaktionen mit Biomolekülen eingehen können.

Die Vernetzer werden üblicherweise in flüssiger Form, d. h. als Lösungen (z. B. wässrige Lösungen), zu der Emulsion hinzugefügt, wobei diese Lösungen den/die Vernetzer vorzugsweise in einer Gesamtkonzentration von 1 bis 40 Gew.-%, insbesondere 5 bis 25 Gew.-% enthalten. Gemäß einer bevorzugten Ausführungsform wird eine 12,5 %ige wässrige Glutaraldehydlösung verwendet.

Der Anteil der zur Emulsion hinzugefügten Vernetzer-Substanz(en) liegt vorzugsweise bei 0,1 bis 10 Vol.-%, insbesondere bei 1 bis 7,5 Vol.-%, jeweils bezogen auf die wässrige Phase.

Des weiteren wird die Vernetzung mittels bifunktionaler Aldehyde, insbesondere Glutaraldehyd, vorzugsweise unter Säurezusatz durchgeführt, weil dadurch die Vernetzungsreaktion erheblich beschleunigt werden kann. Gemäß vorliegender Erfindung wurde überraschenderweise gefunden, dass ein Säurezusatz von 10 Vol.-% oder weniger, vorzugsweise von 5 Vol.-% oder weniger, insbesondere von 3,2 Vol.-% oder weniger, jeweils bezogen auf die wässrige Polymerphase, ausreichend ist, um die durch Glutaraldehyd bewirkte Vernetzung zu beschleunigen und eventuelle Aggregatbildungen des nanopartikulären magnetisierbaren Materials zu verhindern. Die angegebenen Volumenprozentanteile beziehen sich auf 1N bis 3N HCl.

Der Säurezusatz erfolgt vorzugsweise vor dem Emulgierungsschritt, d. h. die Säure wird zur wässrigen Phase hinzugefügt. Alternativ kann der Säurezusatz während des Emulgierens oder nach erfolgter Emulgierung vorgenommen werden.

Neben HCl kommen auch folgende Säuren als Säurezusatz in Betracht: a) Salpetersäure 1-3 N (max. 10 Vol.-% Säurezusatz zur Polymerphase); b) Schwefelsäure 1-3 N (max. 5 Vol.-% Säurezusatz zur Polymerphase); c) HBr; d) Essigsäure; e) Phosphorsäure.

Des weiteren wurde gefunden, dass höhere Säurekonzentrationen (d. h. mehr als 10 Vol.-%) - insbesondere bei höheren Temperaturen (> 60 °C) - zur Zersetzung des nanopartikulären magnetisierbaren Materials, insbesondere des Magnetits, und damit zu einer unerwünschten Freisetzung von Eisenionen führen. Die erfindungsgemäße Verringerung des Säurezusatzes während der Vernetzung kann somit dazu beitragen, dass magnetisierbare Polyvinylalkoholpartikel erhalten werden, die einen hohen Anteil an magnetisierbarem Material, vorzugsweise Magnetit, aufweisen, nämlich mindestens 50 Gew.-% bzw. bis zu 90 Gew.-%.

Die Vernetzungsreaktion ist üblicherweise innerhalb von ca. 10 s bis 5 min nach Zugabe des/der Vernetzer abgeschlossen. Anschließend können die sphärischen, magnetisierbaren Polyvinylalkohol-Mikropartikel mittels bekannter Methoden (z. B. durch Zentrifugation oder magnetische Separation) aus dem flüssigen Reaktionsgemisch abgetrennt werden. Zur Abtrennung eventuell anhaftender Verunreinigungen (z. B. Öl, Emulgatoren) können die Mikropartikel mittels geeigneter Lösemittel bzw. -gemische (z. B. Wasser, Ethanol, Methanol, 2-Propanol, n-Hexan, Aceton, Methylethylketon) gewaschen werden, jeweils durch Resuspendieren und Zentrifugation bzw. magnetische Abtrennung der Partikel.

In einer bevorzugten Ausführungsform umfasst das erfindungsgemäße Verfahren zumindest die folgenden Schritte:
- Dispergieren eines nanopartikulären magnetisierbaren Materials, vorzugsweise Magnetit, in einer wässrigen Phase, die Polyvinylalkohol in gelöster Form enthält, wobei die Polyvinylalkohol-Konzentration in der wässrigen Phase maximal 2,0 Gew.-%, vorzugsweise 0,1 bis 2,0 Gew.-%, besonders bevorzugt 0,5 bis 1,5 Gew.-% beträgt, und wobei die Konzentration des magnetisierbaren Materials bzw. des Magnetits in der wässrigen Phase vorzugsweise 0,5 bis 7,5 Gew.-%, besonders bevorzugt 1 bis 5 Gew.-%, beträgt;
- Hinzufügen der wässrigen Phase zu einer organischen Phase, welche mit der wässrigen Phase nicht mischbar ist und mindestens einen Emulgator enthält, und Erzeugen einer Emulsion durch Rühren bei einer Temperatur von mindestens 50 °C, bevorzugt bei 55 bis 65 °C, insbesondere bei 60 °C;
- Hinzufügen mindestens einer zur Vernetzung von Polyvinylalkohol geeigneten, wasserlöslichen Vernetzer-Substanz unter fortgesetztem Rühren.

In einer weiteren bevorzugten Ausführungsform umfasst das erfindungsgemäße Verfahren zumindest die folgenden Schritte:
- Dispergieren eines nanopartikulären magnetisierbaren Materials, vorzugsweise Magnetit, mit einer Partikelgröße von höchstens 250 nm, vorzugsweise höchstens 100 nm, in einer wässrigen Phase, die Polyvinylalkohol in gelöster Form enthält, wobei die Polyvinylalkohol-Konzentration in der wässrigen Phase maximal 2,0 Gew.-%, vorzugsweise 0,1 bis 2,0 Gew.-%, besonders bevorzugt 0,5 bis 1,5 Gew.-% beträgt, und wobei die Konzentration des magnetisierbaren Materials bzw. des Magnetits in der wässrigen Phase vorzugsweise 0,5 bis 7,5 Gew.-%, besonders bevorzugt 1 bis 5 Gew.-%, beträgt;
- Hinzufügen der wässrigen Phase zu einer organischen Phase, welche mit der wässrigen Phase nicht mischbar ist und mindestens einen Emulgator enthält, und Erzeugen einer Emulsion durch Rühren bei einer Temperatur von mindestens 25 **°**C**,** bevorzugt bei 50 bis 65 °C, insbesondere bei 60 °C;
- Hinzufügen mindestens eines bifunktionalen Aldehyds als Vernetzer unter Zusatz von Salzsäure (1N bis 3N) in einem Volumenanteil von bis zu 5 %, bezogen auf die wässrige Phase.

In einer weiteren bevorzugten Ausführungsform umfasst das erfindungsgemäße Verfahren zumindest die folgenden Schritte:
- Dispergieren eines nanopartikulären magnetisierbaren Materials, vorzugsweise Magnetit, in einer wässrigen Phase, die Polyvinylalkohol in gelöster Form enthält, wobei die Polyvinylalkohol-Konzentration in der wässrigen Phase maximal 2,0 Gew.-%, vorzugsweise 0,1 bis 2,0 Gew.-%, besonders bevorzugt 0,5 bis 1,5 Gew.-% beträgt, und wobei die Konzentration des magnetisierbaren Materials bzw. des Magnetits in der wässrigen Phase vorzugsweise 0,5 bis 7,5 Gew.-%, besonders bevorzugt 1 bis 5 Gew.-%, beträgt;
- Hinzufügen der wässrigen Phase zu einer organischen Phase, welche mit der wässrigen Phase nicht mischbar ist und mindestens einen Emulgator enthält, und Erzeugen einer Emulsion durch Rühren bei einer Temperatur von 25 °C oder höher, vorzugsweise bei 50 bis 65 °C, insbesondere bei 60 °C; wobei die Herstellung der Emulsion mittels eines nach dem Rotor-Stator-Prinzip arbeitenden Dispergierrührers bei einer Rotordrehzahl im Bereich von 500 bis 4000 Umdrehungen/min erfolgt und das Volumen der Emulsion 10 1 oder mehr beträgt;
- Hinzufügen mindestens einer zur Vernetzung von Polyvinylalkohol geeigneten, wasserlöslichen Vernetzer-Substanz unter fortgesetztem Rühren.

In einer besonders bevorzugten Ausführungsform umfasst das erfindungsgemäße Verfahren zumindest die folgenden Schritte:
- Dispergieren eines nanopartikulären magnetisierbaren Materials, vorzugsweise Magnetit, in einer wässrigen Phase, die Polyvinylalkohol in gelöster Form enthält, wobei die Polyvinylalkohol-Konzentration in der wässrigen Phase maximal 2,0 Gew.-%, vorzugsweise 0,1 bis 2,0 Gew.-%, besonders bevorzugt 0,5 bis 1,5 Gew.-% beträgt, und wobei die Konzentration des magnetisierbaren Materials bzw. des Magnetits in der wässrigen Phase vorzugsweise 0,5 bis 7,5 Gew.-%, besonders bevorzugt 1 bis 5 Gew.-%, beträgt;
- Hinzufügen der wässrigen Phase zu einer organischen Phase, welche mit der wässrigen Phase nicht mischbar ist und mindestens einen Emulgator enthält, und Erzeugen einer Emulsion durch Rühren bei einer Temperatur von mindestens 50 **°**C**,** bevorzugt bei 55 bis 65 °C, insbesondere bei 60 °C, wobei die Herstellung der Emulsion mittels eines nach dem Rotor-Stator-Prinzip arbeitenden Dispergierrührers bei einer Rotordrehzahl im Bereich von 500 bis 4000 Umdrehungen/min erfolgt und das Volumen der Emulsion 10 1 oder mehr beträgt;
- Hinzufügen mindestens eines bifunktionalen Aldehyds, vorzugsweise Glutaraldehyd, als Vernetzer unter Zusatz von Salzsäure (1N bis 3N) in einem Volumenanteil von bis zu 10 %, vorzugsweise bis zu 5 %, insbesondere höchstens 3,2 %, jeweils bezogen auf die wässrige Phase.

Jede der vorstehend beschriebenen Ausführungsformen kann mit einem oder mehreren der weiter oben beschriebenen Merkmale kombiniert werden.

Gemäß einer besonders bevorzugten Ausführungsform wird das im ersten Verfahrensschritt der erfindungsgemäßen Herstellungsverfahren eingesetzte nanopartikuläre magnetisierbare Material, vorzugsweise Magnetit, einer Behandlungsmethode unterzogen, welche folgende Schritte umfasst:
- Suspendieren von magnetisierbaren Nanopartikeln in vollentsalztem Wasser mit einem Leitwert von weniger als 100 *µ*S, vorzugsweise weniger als 5 *µ*S/cm;
- Behandeln der wässrigen Nanopartikel-Suspension mittels eines Ultraschall-Homogenisators, vorzugsweise bei einer Ultraschall-Leistung von mindestens 1000 W und einer Beschallungsdauer von mindestens 10 min, vorzugsweise mindestens 0,5; die Ultraschallbehandlung kann vorzugsweise im kontinuierlichen Verfahren (Durchflussverfahren) durchgeführt werden.

Mit den erfindungsgemäßen Verfahren lassen sich sphärische, magnetisierbare Polyvinylalkohol-Partikel mit einer engen Partikelgrößenverteilung im Bereich von 0,5-3 *µ*m beispielsweise in Mengen von mindestens 200 g pro Ansatz gewinnen. Die Ansatzgröße, d. h. das Volumen der Herstellungsemulsion, kann 5 1 oder mehr, insbesondere 10 1 oder mehr betragen, z. B. 50 bis 150 1 oder einige hundert Liter (z. B. 200-500 1).

Optional kann die Partikelgröße durch Verändern eines oder mehrerer der nachfolgenden Parameter beeinflusst werden(unter Beachtung der als erfindungswesentlich angesehenen Grenzwerte, wie in der obigen Beschreibung angegeben):
- Polymerkonzentration (niedrigere Konzentration bewirkt Verringerung der Partikelgröße);
- Rührgeschwindigkeit während des Emulgierens (höhere Geschwindigkeit bewirkt Verringerung der Partikelgröße);
- Auswahl des/der Emulgatoren, sowie Emulgatorkonzentration.

Die mit den erfindungsgemäßen Verfahren hergestellten Polyvinylalkohol-Mikropartikel können ohne weitere Modifizierung zur Bindung von z. B. Biomolekülen verwendet werden, insbesondere durch Bindung an die freien OH-Gruppen des Polyvinylalkohols, oder sie können durch verschiedene, dem Fachmann bekannte Reaktionen aktiviert oder modifiziert werden, um die Bindung von Biomolekülen, Zellen etc. zu ermöglichen. Mit den erwähnten Modifizierungsreaktionen können beispielsweise funktionelle Gruppen oder z. B. Spacermoleküle an die Partikelmatrix oder -Oberfläche gekoppelt werden.

Bevorzugte Beispiele solcher bekannter Aktivierungs- oder Modifizierungsreaktionen sind in WO 97/04862 A1 und den darin zitierten Druckschriften beschrieben. In diesem Zusammenhang ist insbesondere die Aktivierung mittels Aktivierungsreagenzien wie Bromcyan (zur Kopplung von Liganden mit primären Aminogruppen, z. B. Antikörper), Epichlorhydrin, 1,1'-Carbonyldiimidazol oder Hexamethylendiisocyanat zu nennen.

Die Einführung von Spacermolekülen kann auf an sich bekannte Weise durch Pfropfpolymerisation von Vinylmonomeren (insbesondere Acrylmonomeren) unter katalytischer Wirkung von Cer(IV)-Salzen erfolgen, wie in WO 97/04862 A1 und den darin zitierten Druckschriften beschrieben.
Die an die magnetisierbaren Polyvinylalkohol-Mikropartikel gekoppelten Spacer können sodann beispielsweise zur Bindung von Biomolekülen verwendet werden.

Die Erfindung bezieht sich ferner auf sphärische, magnetisierbare Polyvinylalkohol-Mikropartikel mit einer Partikelgrößenverteilung im Bereich von 0,5 bis 3 *µ*m, erhältlich nach einem der oben beschriebenen erfindungsgemäßen Herstellungsverfahren. Vorzugsweise weisen die Mikropartikel eine Größenverteilung im Bereich von 0,8 bis 3 *µ*m oder insbesondere im Bereich von 0,5 bis 1 *µ*m auf. Gemäß einer weiteren, bevorzugten Ausführungsform weisen die Mikropartikel eine Größenverteilung im Bereich von 1,25 bis 2,25 *µ*m auf.

Dabei bedeutet die Angabe "Größenverteilung im Bereich von ...", dass mindestens 75%, vorzugsweise mindestens 90%, besonders bevorzugt mindestens 95 % der Partikel eine Partikelgröße aufweisen, die innerhalb des jeweils angegebenen Größenbereichs liegt.

Die Partikelgrößenverteilung der erfindungsgemäßen Mikropartikel kann als monomodal bezeichnet werden, d. h. sie weist im wesentlichen nur ein einziges Maximum auf.

Die Partikelgröße kann auf dem Fachmann bekannte Weise bestimmt werden, beispielsweise mittels DLS/PCS (Dynamische Lichtstreuung / Photonen-Korrelations-Spektroskopie). Hierfür geeignete Messgeräte sind im Handel erhältlich (z. B. Beckman Coulter DelsaNano C, D-47807 Krefeld).

Die verwendete PCS-Methode liefert als Ergebnis die mittlere Partikelgröße der gemessenen Probe und einen dazugehörigen Polydispersitätsindex (PDI), der ein Maß für die Breite der Partikelgrößenverteilung darstellt. Bei den mit den erfindungsgemäßen Verfahren hergestellten magnetisierbaren Mikropartikel, welche eine enge Größenverteilung aufweisen, liegt der Polydispersitätsindex (PDI) vorzugsweise bei oder unterhalb von 0,25, insbesondere zwischen 0,1 und 0,25.

Gemäß einer bevorzugten Ausführungsform zeichnen sich die erfindungsgemäßen magnetisierbaren Mikropartikel dadurch aus, dass der Gehalt an nanopartikulärem Material, insbesondere Magnetit, mindestens 50 Gew.-%, vorzugsweise mindestens 60 Gew.-%, stärker bevorzugt mindestens 75 Gew.-%, und insbesondere bis zu 90 Gew.-%, beträgt. Dies lässt sich erfindungsgemäß insbesondere dadurch erreichen, dass die Polyvinylalkohol-Konzentration in der wässrigen Phase auf maximal 1,5 Gew.-% begrenzt wird, wie oben beschrieben. Aufgrund des erhöhten Magnetit-Gehalts und wegen der engen Partikelgrößenverteilung (wie oben angegeben) zeichnen sich diese magnetisierbaren Mikropartikel durch verbesserte Separationseigenschaften im Magnetfeld aus.

Die Bestimmung des Anteils an magnetisierbarem anorganischem Material kann auf dem Fachmann bekannte Weise z. B. mittels Verbrennungsanalyse erfolgen.

Aufgrund des hohen Magnetitgehalts der erfindungsgemäßen magnetisierbaren Mikropartikel können auch bei verringerter Partikelgröße (z. B. im Bereich von 0,5 bis 1 *µ*m) noch gute magnetische Separationseigenschaften gewährleistet werden. Besonders bevorzugte erfindungsgemäße Partikel sind deshalb solche, die eine Partikelgrößenverteilung im Bereich von 0,5 bis 1 *µ*m und einen Magnetitgehalt von 50 bis 90 Gew.-%, vorzugsweise 50 bis 75 Gew.-%, besonders bevorzugt 50 bis 60 Gew.-% aufweisen.

Die mit den erfindungsgemäßen Verfahren erhältlichen oder hergestellten magnetisierbaren Mikropartikel zeichnen sich trotz ihres erhöhten Magnetitgehaltes (mind. 50 Gew.-%, vorzugsweise 50 bis 60 Gew.-%; besonders bevorzugt mindestens 60 Gew.-%) durch eine deutliche Reduzierung der unspezifischen Bindung (insbesondere von Proteinen) bei der Verwendung in den erwähnten Nachweisverfahren, insbesondere bei der PCR, aus. Diese Reduzierung der unspezifischen Bindung ist darauf zurückzuführen, dass mit den erfindungsgemäßen Herstellungsverfahren eine besonders effiziente Einkapselung des magnetisierbaren Materials (z. B. Magnetit) erreicht wird. Infolgedessen ist der Anteil an freiem Magnetit (bzw. nanopartikulären magnetisierbaren Materials), der für unspezifische Bindungen verantwortlich ist, äußerst gering.

Die effizientere Einkapselung des Magnetits bzw. die Verringerung des Anteils an freiem Magnetit ist anhand einer messbaren Veränderung des Zetapotentials der Partikel, verglichen mit herkömmlichen Partikeln (z. B. WO 97/04862 A1), nachweisbar (siehe Beispiel 3 und Tabelle 1). Die mit den erfindungsgemäßen Verfahren hergestellten oder erhältlichen Partikel weisen vorzugsweise ein um mindestens 15 %, vorzugsweise um 25 %, insbesondere um 30 % erhöhtes Zetapotential auf (bezogen auf den Absolutwert des Zetapotentials). Es wird angenommen, dass die beobachtete Veränderung des Zetapotentials auf die Verringerung des an der Partikeloberfläche vorhandenen freien Magnetits zurückzuführen ist (bei gleichbleibender Partikelgröße und -Oberfläche), und dass das Zetapotential somit als ein Maß für die Effizienz der Einkapselung des Magnetits angesehen werden kann. Vorzugsweise ist das Zetapotential der magnetischen Mikropartikel ≤ -35 mV, besonders bevorzugt ≤ -40 mV.

Die vorliegende Erfindung umfasst deshalb sphärische, magnetisierbare Polyvinylalkohol-Mikropartikel mit einem Gehalt an nanopartikulärem magnetisierbaren Material, wobei die Mikropartikel ein Zetapotential von ≤ -35 mV, besonders insbesondere von ≤ -40 mV aufweisen.
Die Partikelgrößenverteilung liegt vorzugsweise im Bereich von 0,5 bis 3 *µ*m, und besonders bevorzugt im Bereich von 0,5 bis 1 *µ*m oder im Bereich von 1,25 bis 2,25 *µ*m.
Der Gehalt an nanopartikulärem magnetisierbaren Material, insbesondere Magnetit, beträgt vorzugsweise mindestens 50 Gew.-%, stärker bevorzugt mindestens 60 Gew.-%, besonders bevorzugt mindestens 75 Gew.-%, wobei dieser Gehalt bis zu 90 Gew.-% betragen kann.

Die mit den erfindungsgemäßen Verfahren erhältlichen oder hergestellten magnetisierbaren Mikropartikel zeichnen sich ferner dadurch aus, dass sie bei PCR-Anwendungen einen deutlich verringerten inhibitorischen Einfluss auf die PCR-Reaktion haben oder dass ein solcher Einfluss völlig fehlt, im Vergleich zu vorbekannten Mikropartikeln (siehe Beispiel 4 und Tabellen 3 u. 4). Die Anwesenheit von inhibitorischen Komponenten im PCR-Reaktionsansatz führt zu einer Verzögerung der Amplifikationsreaktion und dadurch zu einer Erhöhung des Ct-Wertes. Mit diesem Wert wird bei der "Real Time Quantitative PCR" die Anzahl der Zyklen bezeichnet, die benötigt werden, bis das Fluoreszenzsignal den Schwellenwert überschreitet und die exponentielle Phase der PCR beginnt. Da sich der Ct-Wert umgekehrt proportional zur Menge der in der Probe enthaltenen Nukleinsäure verhält, führt die Anwesenheit von inhibitorischen Komponenten (wie z. B. Mikropartikel mit inhibitorischer Wirkung) zu einer Verfälschung der Ergebnisse.

Überraschenderweise wurde gefunden, dass bei Verwendung der mit den erfindungsgemäßen Verfahren erhältlichen oder hergestellten magnetisierbaren Mikropartikel in PCR-Anwendungen der Ct-Wert unverändert bleibt oder um maximal 2 %, vorzugsweise um maximal 1 %, besonders bevorzugt um maximal 0,5 % erhöht wird.
Somit umfasst die vorliegende Erfindung sphärische, magnetisierbare Polyvinylalkohol-Mikropartikel mit einem Gehalt an nanopartikulärem magnetisierbaren Material, wobei die Mikropartikel beim Einsatz in PCR-Verfahren keine Veränderung des Ct-Wertes oder nur eine geringfügige Erhöhung des Ct-Wertes, vorzugsweise um maximal 2 %, besonders bevorzugt um maximal 1 %, insbesondere um maximal 0,5 %, bewirken.

Die Partikelgrößenverteilung liegt vorzugsweise im Bereich von 0,5 bis 3 *µ*m, und besonders bevorzugt im Bereich von 0,5 bis 1 *µ*m oder im Bereich von 1,25 bis 2,25 *µ*m. Der Gehalt an nanopartikulärem magnetisierbaren Material, insbesondere Magnetit, beträgt vorzugsweise mindestens 50 Gew.-%, stärker bevorzugt mindestens 60 Gew.-%, besonders bevorzugt mindestens 75 Gew.-%, wobei dieser Gehalt bis zu 90 Gew.-% betragen kann.

Die erfindungsgemäßen magnetisierbaren Mikropartikel weisen funktionelle Gruppen auf, die eine Kopplung von Biomolekülen oder Liganden über kovalente oder nichtkovalente Bindungen ermöglichen. Als funktionelle Gruppen kommen die Hydroxylgruppen der Polyvinylalkohol-Matrix, aber auch durch nachträgliche Modifikationsreaktionen eingeführte Gruppen - wie oben erwähnt - in Betracht. Gemäß einer bevorzugten Ausführungsform sind die funktionellen Gruppen über Spacermoleküle an Polyvinylalkohol gebunden.

Als Biomoleküle oder Liganden, welche kovalent oder nichtkovalent an die magnetisierbaren Mikropartikel gebunden werden können, kommen insbesondere Antikörper, Avidin, Biotin, Protein A, Protein G, Lektine, Oligosaccharide, Oligonucleotide, Enzyme, Enzyminhibitoren, Enzymsubstrate, Rezeptorproteine, Albumin, Gelatine, Glutathion, Aminosäuren, Peptide, Hormone und/oder Neurotransmitter in Betracht. Darüber hinaus kommen grundsätzlich alle weiteren Biomoleküle oder Liganden in Betracht, die im Stand der Technik bei der Affinitätschromatographie oder für diagnostische Zwecke Verwendung finden.

Die erfindungsgemäßen sphärischen, magnetisierbaren Polyvinylalkohol-Mikropartikel sind aufgrund ihres hohen Gehalts (mind. 50 Gew.-%) an magnetisierbarem nanopartikulären Material, vorzugsweise Magnetit, und aufgrund ihrer engen Partikelgrößenverteilung im Bereich von 0,5 bis 3 *µ*m insbesondere für die Verwendung bei automatisierten Separationsverfahren geeignet, insbesondere für die automatisierte Nukleinsäure-Aufreinigung. Aufgrund der engen Größenverteilung wird die vollständige Separation der magnetisierbaren Mikropartikel insbesondere bei der automatisierten Nukleinsäureaufreinigung vereinfacht.

Des weiteren können mit den erfindungsgemäßen sphärischen, magnetisierbaren Polyvinylalkohol-Mikropartikeln, deren Gehalt an magnetisierbarem Material mehr als 50 Gew.-% und bis zu 90 Gew.-% betragen kann, trotz der verringerten bzw. geringen Partikelgröße sehr gute magnetische Separationseigenschaften erzielt werden (d. h. schnelle, vollständige und reproduzierbare Separation mit hohen Ausbeuten). Dadurch eignen sich diese erfindungsgemäßen Mikropartikel auch in besonderer Weise für die Behandlung größerer Probenvolumina (z. B. Isolierung von genomischer DNA aus 10 ml Vollblut).

Darüber hinaus eignen sich die erfindungsgemäßen Polyvinylalkohol-Mikropartikel auch vorzüglich zur Behandlung sehr kleiner Probenvolumina (z. B. 10 *µ*l), da aufgrund des niedrigen Partikeldurchmessers und der damit verbundenen großen aktiven Gesamtoberfläche eine kleine Masse an Partikeln zur Aufreinigung der jeweiligen Probe ausreichend ist.

Die erfindungsgemäßen Polyvinylalkohol-Mikropartikel können beispielsweise zur Isolierung, Reinigung oder Anreicherung von Zellen, Nukleinsäuren, Peptiden, Proteinen, Toxinen, Viren, Bakterien, Antikörpern, Enzymen, Antigenen oder Rezeptoren verwendet werden. Bevorzugte Anwendungsgebiete betreffen diagnostische oder forensische Nachweisverfahren, in Immunoassays, DNA-Sequenzierung, PCR-Produkt-Reinigung, Zellfraktionierung, Protein-Isolierung, Affinitätsreinigung, Immunpräzipitation, Gewebetypisierung, Oligonukleotidsynthese oder Peptidsynthese.

Durch die vorliegende Erfindung werden magnetisierbare Mikropartikel mit verbesserten Separationseigenschaften bereitgestellt, die insbesondere für automatisierte Separationsverfahren geeignet sind. Darüber hinaus wird durch die erfindungsgemäßen Herstellungsverfahren die Produktion dieser Partikel in großem Maßstab und mit hoher Chargen-Konsistenz ermöglicht.

### Beispiele

### Beispiel 1: Herstellung einer Magnetit-Kolloidsuspension

Magnetit-Kolloid: Das nanokristalline Magnetit wird wie oben beschrieben mittels bekannter Verfahren hergestellt. Danach wird die Suspension durch wiederholtes Zentrifugieren bei 3000 x g für jeweils fünf Minuten und erneutes Resuspendieren in demineralisiertem Wasser bis zu einem Leitwert der Suspension von < 500 *µ*S/cm salzfrei gewaschen. Das gemäß vorliegender Erfindung hergestellte Magnetit hat ein schwach negatives, nahezu neutrales Zetapotential (im Bereich von ca. -4,5 bis -0,5 mV).

180 g des so gewonnenen Magnetits werden in 4400 ml demineralisiertem Wasser suspendiert und für 2 Stunden in einem In-Line Ultraschallverfahren (Durchflussverfahren) mit mindestens 1000 W Leistung behandelt (Dr. Hielscher Sonopuls 2000 W; Hielscher Ultrasonics GmbH, D-14513 Teltow).

Danach werden 600 ml einer 12,5 %igen PVA Lösung (Gew.-%) hinzugegeben und weitere zwei Stunden beschallt. Anschließend wird die Suspension bei 2000 x g für 10 Minuten zentrifugiert. Es wird eine PVA-haltige Magnetitsupension mit einem Größenmaximum von 100 nm und einem Magnetitgehalt von 2,8 %, sowie 1,4% PVA gewonnen. Die Bestimmung der Partikelgröße erfolgt mittels PCS (Beckman Coulter DelsaNano C) .

### Beispiel 2: Herstellung von magnetisierbaren Polyvinylalkohol-Mikropartikeln

5000 ml des Magnetitkolloids gemäß Beispiel 1 werden mit 660 ml HCl (2,5 M) innig gemischt. Diese Suspension wird zu 60 1 eines handelsüblichen Pflanzenöls, das 1,5 Vol.-% TWEEN^{®}, 0,5 Vol.-% Sorbitansesquioleat, 2,5 Vol.-% Dehymuls^{®} HRE und 0,75 Vol.-% Hypermer^{®} enthält, gegeben, nachdem die org. Phase auf 60°C aufgeheizt wurde.

Anschließend wird für 10 Minuten mit einem Dispergierrührer (Ystral Dispermix) bei 60 °C und bei einer Rührgeschwindigkeit von 3500 U/Min gerührt. Nach Zugabe von 550 ml einer 12,5 %igen (in Wasser) Glutaraldehydlösung wird für weitere 35 Min gerührt (Ystral Dispermix, 3500 U/Min).
Die Partikel werden magnetisch von der Suspension abgetrennt und wiederholt mit Wasser (keine organischen Lösungsmittel) gewaschen. Es werden 250 g Magnetpartikel mit einer Größe von 1-3 *µ*m und einem Magnetitgehalt von 55 Gew.-% gewonnen. Der Polydispersitätsindex (PDI) liegt zwischen 0,1 und 0,25. Die Bestimmung der Partikelgröße und des PDI erfolgte mittels PCS (Beckman Coulter DelsaNano C).

### Beispiel 3: Bestimmung des Zetapotentials

Die erfindungsgemäßen magnetisierbaren Mikropartikel zeichnen sich unter anderem dadurch aus, dass sie bei biochemischen Nachweisverfahren (insbesondere bei der PCR) weniger unspezifische Bindungen, Verschleppung von störenden Bestandteilen etc. verursachen, als dies bei den im Stand der Technik beschriebenen Partikeln der Fall ist. Es wird angenommen, dass diese vorteilhafte Eigenschaft gemäß vorliegender Erfindung durch eine verbesserte Einkapselung des magnetisierbaren Materials (Magnetit) bewirkt wird, so dass weniger freies, d. h. nicht eingekapseltes Magnetit an der Partikeloberfläche exponiert ist.
Da durch die Anwesenheit von nicht eingekapseltem Magnetit die Oberflächeneigenschaften der Partikel verändert werden, ist anzunehmen, dass diese Veränderung durch die Messung des Zetapotentials - welches im wesentlichen proportional zur Anzahl der Oberflächenladungen ist - nachgewiesen werden kann. Diese Hypothese wurde wie folgt überprüft:

Es wurden erfindungsgemäße Polyvinylalkohol-Mikropartikel hergestellt, wie in Beispiel 2 beschrieben. Für Vergleichszwecke wurden magnetische Polyvinylalkohol-Partikel gemäß WO 97/04862 A1, Beispiel 2, hergestellt. In beiden Fällen lag die Partikelgröße im Bereich von 1-3 *µ*m. Anschließend wurde mit Proben aus mehreren Partikelchargen eine Messung des Zetapotentials durchgeführt. Die Ergebnisse sind in der nachfolgende Tabelle 1 dargestellt:

**Tabelle 1:**

| *Magnetpartikel gemäß WO 97*/*04862* | | | | *Erfindungsgemäße Magnetpartikel* | | |
|---|---|---|---|---|---|---|
| | Chargen-Nr. | Zetapotential [mV] | | | Chargen-Nr. | Zetapotential [mV] |
| 1 | MP121 | - 29,41 | | 1 | C348 | - 39,59 |
| 2 | MP121 | - 30,91 | | 2 | C349 | - 39,36 |
| 3 | C238 | - 30,38 | | 3 | C350 | - 39,38 |
| 4 | C257 | - 27,33 | | 4 | C351 | - 40,12 |
| 5 | C271 | - 26,83 | | 5 | C352 | - 39,75 |
| 6 | C283 | - 28,74 | | | | |
| Ø | | **- 28,93** | | Ø | | **- 39,64** |

Die nach dem erfindungsgemäßen Verfahren hergestellten Partikel weisen ein durchschnittliches Zetapotential von ca. - 40 mV auf, welches im Vergleich zu den gemäß WO 97/04862 hergestellten Partikeln um 30 % erhöht ist (bezogen auf den Absolutbetrag).

Dieser Befund bestätigt die Annahme, dass bei den erfindungsgemäßen Partikeln der Anteil des freien Magnetits verringert werden konnte. Je geringer der Anteil an freiem Magnetit an der Gesamtoberfläche ist und je größer der von Polyvinylalkohol bedeckte Anteil der Gesamtoberfläche ist, desto mehr potentiell oxidierbare funktionelle Gruppen (und damit potentielle Ladungsträger) sind auf der Oberfläche vorhanden. Dies äußert sich in einer entsprechenden Änderung des Zetapotentials, wie in Tab. 1 dargestellt. Da der Partikeldurchmesser und damit die Gesamtoberfläche der Partikel stets konstant war (1-3 *µ*m), ist davon auszugehen, dass die beobachtete Veränderung des Zetapotentials auf die Verringerung des Anteils an freiem Magnetit bei den erfindungsgemäßen Partikeln zurückzuführen ist.

Diese Annahme wird durch zusätzliche Experimente bestärkt, in denen gezeigt werden konnte, dass das Zetapotential der magnetischen Polymerpartikel wieder abgesenkt werden kann (bezogen auf den Absolutbetrag), indem die Partikel mit nanokristallinem Magnetit inkubiert werden. Hierbei bindet das Magnetit über Van-der-Waals-Wechselwirkungen an die Partikeloberfläche und bedeckt damit einen Teil der Partikeloberfläche, so dass die darunterliegenden Ladungen abgeschirmt bzw. unzugänglich werden. Dies hat erwartungsgemäß eine Absenkung des Zetapotentials (bezogen auf den Absolutbetrag) zur Folge, wie in der nachfolgenden Tabelle 2 dargestellt:

**Tabelle 2:**

| | Chargen-Nr. | Zetapotential [mV] |
|---|---|---|
| 1 | C348 | - 39,59 |
| 2 | C348 + M | - 32,28 |
| 3 | C349 | - 39,36 |
| 4 | C349 + M | - 34,1 |
| 5 | C350 | - 39,38 |
| 6 | C350 + M | - 33,13 |
| 7 | C351 | - 40,12 |
| 8 | C351 + M | - 33,14 |
| 9 | C352 | - 39,75 |
| 10 | C352 + M | - 32,98 |

Für die in Tab. 2 dargestellten Zetapotential-Messungen wurden nach dem erfindungsgemäßen Verfahren hergestellte Partikel verwendet (wie in Tab. 1 bzw. Beispiel 2). Die Angabe "+ M" bedeutet, dass die jeweiligen Proben mit freiem Magnetit inkubiert wurden.

Aufgrund des stärker negativen Zetapotentials neigen die erfindungsgemäßen magnetisierbaren Polyvinylalkoholpartikel in geringerem Maße zur Bildung von Agglomeraten bzw. zur Agglutination als dies bei den vorbekannten Polyvinylalkohol-Partikeln (WO 97/04862 A1) der Fall ist.

### Beispiel 4: Verhalten der erfindungsgemäßen magnetisierbaren Polyvinylalkohol-Mikropartikel beim Einsatz in der PCR

Es wurde untersucht, inwieweit die Anwesenheit von magnetisierbaren Polyvinylalkohol-Mikropartikeln in PCR-Proben einen inhibitorischen Einfluss auf die PCR ausübt. Hierbei wurden erfindungsgemäße Mikropartikel (s. Beispiele 2 und 3) mit herkömmlichen Partikeln (WO 97/04862 A1; s. Beispiel 3) verglichen.

Die in der PCR eingesetzte Template-DNA wurde gewonnen, indem humanes Plasma mit einem definierten Phagentiter des Bakteriophagen Phi X 174 gespiked und hieraus die Nukleinsäuren extrahiert wurden. Anschließend wurden die Nukleinsäuren mittels Real Time PCR amplifiziert. Zur Überprüfung möglicher inhibitorischer Einflüsse der magnetisierbaren Polymerpartikel wurden die Amplifikationsreaktionen mit den zu untersuchenden magnetisierbaren Polyvinylalkohol-Mikropartikeln versetzt, wobei jeweils 25 *µ*g, 50 *µ*g oder 100 *µ*g Partikel pro Reaktionsansatz zugegeben wurden (s. Tabellen 3 u. 4).

In den nachfolgenden Tabellen 3 und 4 sind die mittels Real Time PCR ermittelten Ct-Werte aufgelistet. Der Ct-Wert gibt die Anzahl der Zyklen an, die benötigt werden, bis das Fluoreszenzsignal den Schwellenwert überschreitet. Bei der Positivkontrolle (PCR-Reaktion ohne Zugabe von magnetisierbaren Polymerpartikeln) betrug der Ct-Wert 25,00.

**Tabelle 3: Erfindungsgemäße magnetisierbare Partikel: Ct-Werte (Real Time PCR)**

| | 1 | 2 | 3 | 4 | 5 | Ø |
|---|---|---|---|---|---|---|
| 25 µg | 25,00 | 25,00 | 24,72 | 25,00 | 24,69 | 24,88 |
| 50 µg | 25,00 | 25,02 | 25,07 | 25,14 | 25,10 | 25,07 |
| 100 µg | 25,09 | 25,16 | 25,29 | 25,06 | 25,09 | 25,14 |

Die Angaben in den Spalten 1 bis 5 beziehen sich auf unterschiedliche Chargen der erfindungsgemäßen Partikel.
Wie aus Tabelle 3 ersichtlich, bleibt der Ct-Wert unverändert bei 25. Dies bedeutet, dass die Amplifikation der Nukleinsäuren durch die in der Reaktion vorhandenen Partikel nicht gehemmt wird.

**Tabelle 4: Magnetisierbare Partikel gemäß Stand der Technik (WO 97/04862 A1): Ct-Werte (Real Time PCR)**

| | 1 | 2 | 3 | 4 | 5 | Ø |
|---|---|---|---|---|---|---|
| 25 µg | 26,29 | 26,24 | 26,14 | 26,24 | 26,2 | 26,22 |
| 50 µg | 26,74 | 26,68 | 26,7 | 26,69 | 26,57 | 26,68 |
| 100 µg | 28 | 28,2 | 28,16 | 28,24 | 28 | 28,12 |

Die Angaben in den Spalten 1 bis 5 beziehen sich auf unterschiedliche Chargen der gemäß WO 97/04862 A1 hergestellten Partikel.

Im Unterschied zu Tabelle 3 (erfindungsgemäße Partikel) führte die Zugabe der vorbekannten Partikel zur PCR zu einer Erhöhung der Ct-Werte, abhängig von der zugesetzten Partikelmenge (25 / 50 / 100 *µ*g). Durch Zugabe von 100 *µ*g wurde der Ct-Wert auf 28 erhöht. Die Erhöhung des Ct-Wertes bedeutet eine Verzögerung bei der Entstehung der Fluoreszenzsignale, d. h. eine verzögerte Amplifikation der DNA. Diese Verzögerung zeigt somit den inhibitorischen Einfluss der herkömmlichen Magnetpartikel (WO 97/04862 A1) an, im Unterschied zu den erfindungsgemäßen Partikeln, die keinen derartigen inhibitorischen Effekt zeigen (Tab. 3).

## Patentansprüche

1. Verfahren zur Herstellung von sphärischen, magnetisierbaren Polyvinylalkohol-Mikropartikeln mit einer Partikelgrößenverteilung im Bereich von 0,5 bis 3 µm, umfassend folgende Schritte:
- Dispergieren eines nanopartikulären magnetisierbaren Materials, vorzugsweise Magnetit, in einer wässrigen Phase, die Polyvinylalkohol in gelöster Form enthält;
- Hinzufügen der wässrigen Phase zu einer organischen Phase, welche mit der wässrigen Phase nicht mischbar ist und mindestens einen Emulgator enthält, und Erzeugen einer Emulsion durch Rühren bei einer Temperatur von 40 °C oder höher;
- Hinzufügen mindestens einer zur Vernetzung von Polyvinylalkohol geeigneten, wasserlöslichen Vernetzer-Substanz unter fortgesetztem Rühren.

2. Verfahren nach Anspruch 1, **dadurch gekennzeichnet, dass** das nanopartikuläre magnetisierbare Material aus magnetisierbaren Nanopartikeln mit einer Partikelgröße im Bereich von 5 bis 250 nm, vorzugsweise 5 bis 100 nm, besonders bevorzugt 5 bis 50 nm besteht.

3. Verfahren nach Anspruch 1 oder 2, **dadurch gekennzeichnet, dass** als nanopartikuläres magnetisierbares Material nano-kristalliner Magnetit verwendet wird.

4. Verfahren nach einem der vorangehenden Ansprüche, **dadurch gekennzeichnet, dass** die wässrige Phase vor dem Hinzufügen zu der organischen Phase einer Behandlung unterzogen wird, durch welche Partikel mit einer Größe von > 250 nm, insbesondere mit einer Größe von > 100 nm entfernt werden, vorzugsweise mittels Zentrifugation.

5. Verfahren nach einem der vorangehenden Ansprüche, **dadurch gekennzeichnet, dass** die Polyvinylalkohol-Konzentration in der wässrigen Phase maximal 2,0 Gew.-%, vorzugsweise 0,1 bis 2,0 Gew.-%, besonders bevorzugt 0,5 bis 1,5 Gew.-% beträgt.

6. Verfahren nach einem der vorangehenden Ansprüche, **dadurch gekennzeichnet, dass** die Konzentration des nanopartikulären magnetisierbaren Materials in der wässrigen Phase 0,5 bis 7,5 Gew.-%, vorzugsweise 1 bis 5 Gew.-%, beträgt.

7. Verfahren nach einem der vorangehenden Ansprüche, **dadurch gekennzeichnet, dass** die organische, nicht mit Wasser mischbare Phase eine oder mehrere aus der pflanzliche Öle, synthetische Öle, Mineralöle, Silikonöle und Paraffinöle umfassenden Gruppe ausgewählte Substanz(en) umfasst.

8. Verfahren nach einem der vorangehenden Ansprüche, **dadurch gekennzeichnet, dass** der Emulgator-Anteil in der organischen Phase 0,1 bis 10 Gew.-%, vorzugsweise 1 bis 7,5 Gew.-% beträgt.

9. Verfahren nach einem der vorangehenden Ansprüche, **dadurch gekennzeichnet, dass** die Vernetzer-Substanz aus der bifunktionale Aldehyde, insbesondere Glutaraldehyd, sowie Säurechloride und Divinylsulfon umfassenden Gruppe ausgewählt ist.

10. Verfahren nach einem der vorangehenden Ansprüche, **dadurch gekennzeichnet, dass** die Vernetzer-Substanz in flüssiger Form zugesetzt wird, wobei der Anteil 0,1 bis 10 Vol.-%, vorzugsweise 1 bis 7,5 Vol.-% beträgt, jeweils bezogen auf die wässrige Phase.

11. Verfahren nach einem der vorangehenden Ansprüche, **dadurch gekennzeichnet, dass** als Vernetzer-Substanz(en) bifunktionale Aldehyde verwendet werden und die Vernetzungsreaktion unter Zusatz einer Säure durchgeführt wird, wobei vorzugsweise 1N bis 3N Salzsäure in einem Volumenanteil von bis zu 10 %, vorzugsweise bis zu 5 %, insbesondere höchstens 3,2 %, bezogen auf die wässrige Phase, verwendet wird.

12. Verfahren nach einem der vorangehenden Ansprüche, **dadurch gekennzeichnet, dass** das Erzeugen der Emulsion durch Rühren bei einer Temperatur von mindestens 50 °C, besonders bevorzugt bei 55 bis 65 °C erfolgt.

13. Verfahren nach einem der vorangehenden Ansprüche, **dadurch gekennzeichnet, dass** die Herstellung der Emulsion mittels eines nach dem Rotor-Stator-Prinzip arbeitenden Dispergierrührers erfolgt.

14. Verfahren nach einem oder mehreren der vorangehenden Ansprüche, **gekennzeichnet durch** folgende Schritte:
- Dispergieren eines nanopartikulären magnetisierbaren Materials, vorzugsweise Magnetit, in einer wässrigen Phase, die Polyvinylalkohol in gelöster Form enthält, wobei die Polyvinylalkohol-Konzentration in der wässrigen Phase maximal 2,0 Gew.-%, vorzugsweise 0,1 bis 2,0 Gew.-%, besonders bevorzugt 0,5 bis 1,5 Gew.-% beträgt;
- Hinzufügen der wässrigen Phase zu einer organischen Phase, welche mit der wässrigen Phase nicht mischbar ist und mindestens einen Emulgator enthält, und Erzeugen einer Emulsion **durch** Rühren bei einer Temperatur von mindestens 40 °C, bevorzugt bei 55 bis 65 °C, insbesondere bei 60 °C erfolgt.
- Hinzufügen mindestens einer zur Vernetzung von Polyvinylalkohol geeigneten, wasserlöslichen Vernetzer-Substanz unter fortgesetztem Rühren.

15. Verfahren nach einem oder mehreren der vorangehenden Ansprüche, **gekennzeichnet durch** folgende Schritte:
- Dispergieren eines nanopartikulären magnetisierbaren Materials, vorzugsweise Magnetit, mit einer Partikelgröße von höchstens 250 nm, vorzugsweise höchstens 100 nm, in einer wässrigen Phase, die Polyvinylalkohol in gelöster Form enthält, wobei die Polyvinylalkohol-Konzentration in der wässrigen Phase maximal 2,0 Gew.-%, vorzugsweise 0,1 bis 2,0 Gew.-%, besonders bevorzugt 0,5 bis 1,5 Gew.-% beträgt;
- Hinzufügen der wässrigen Phase zu einer organischen Phase, welche mit der wässrigen Phase nicht mischbar ist und mindestens einen Emulgator enthält, und Erzeugen einer Emulsion **durch** Rühren bei einer Temperatur von mindestens 40 °C, bevorzugt bei 50 bis 65 °C, insbesondere bei 60 °C erfolgt.
- Hinzufügen mindestens eines bifunktionalen Aldehyds als Vernetzer unter Zusatz von 1N bis 3N Salzsäure in einem Volumenanteil von bis zu 10 %, vorzugsweise bis zu 5 %, insbesondere höchstens 3,2 %, jeweils bezogen auf die wässrige Phase.

16. Verfahren nach einem oder mehreren der vorangehenden Ansprüche, **gekennzeichnet durch** folgende Schritte:
- Dispergieren eines nanopartikulären magnetisierbaren Materials, vorzugsweise Magnetit, in einer wässrigen Phase, die Polyvinylalkohol in gelöster Form enthält, wobei die Polyvinylalkohol-Konzentration in der wässrigen Phase maximal 2,0 Gew.-%, vorzugsweise 0,1 bis 2,0 Gew.-%, besonders bevorzugt 0,5 bis 1,5 Gew.-% beträgt;
- Hinzufügen der wässrigen Phase zu einer organischen Phase, welche mit der wässrigen Phase nicht mischbar ist und mindestens einen Emulgator enthält, und Erzeugen einer Emulsion **durch** Rühren bei einer Temperatur von 40 °C oder höher, wobei die Herstellung der Emulsion mittels eines nach dem Rotor-Stator-Prinzip arbeitenden Dispergierrührers bei einer Rotordrehzahl im Bereich von 500 bis 4000 Umdrehungen/min erfolgt und das Volumen der Emulsion 10 l oder mehr beträgt;
- Hinzufügen mindestens einer zur Vernetzung von Polyvinylalkohol geeigneten, wasserlöslichen Vernetzer-Substanz unter fortgesetztem Rühren.

17. Verfahren nach einem oder mehreren der vorangehenden Ansprüche, **gekennzeichnet durch** folgende Schritte:
- Dispergieren eines nanopartikulären magnetisierbaren Materials, vorzugsweise Magnetit, in einer wässrigen Phase, die Polyvinylalkohol in gelöster Form enthält, wobei die Polyvinylalkohol-Konzentration in der wässrigen Phase maximal 2,0 Gew.-%, vorzugsweise 0,1 bis 2,0 Gew.-%, besonders bevorzugt 0,5 bis 1,5 Gew.-% beträgt;
- Hinzufügen der wässrigen Phase zu einer organischen Phase, welche mit der wässrigen Phase nicht mischbar ist und mindestens einen Emulgator enthält, und Erzeugen einer Emulsion **durch** Rühren bei einer Temperatur von mindestens 40 °C, bevorzugt bei 55 bis 65 °C, insbesondere bei 60 °C erfolgt, wobei die Herstellung der Emulsion mittels eines nach dem Rotor-Stator-Prinzip arbeitenden Dispergierrührers bei einer Rotordrehzahl im Bereich von 500 bis 4000 Umdrehungen/min erfolgt und das Volumen der Emulsion 10 1 oder mehr beträgt;
- Hinzufügen mindestens eines bifunktionalen Aldehyds, vorzugsweise Glutaraldehyd, als Vernetzer unter Zusatz von 1N bis 3N Salzsäure in einem Volumenanteil von bis zu 10 %, vorzugsweise bis zu 5 %, insbesondere höchstens 3,2 %, jeweils bezogen auf die wässrige Phase.

18. Verfahren nach einem der vorangehenden Ansprüche, **dadurch gekennzeichnet, dass** das verwendete nanopartikuläre magnetisierbare Material, vorzugsweise Magnetit, einem Behandlungsverfahren unterzogen wird, das folgende Schritte aufweist:
- Suspendieren von magnetisierbaren Nanopartikeln in vollentsalztem Wasser mit einem Leitwert von weniger als 100 µS/cm, vorzugsweise weniger als 5 µS/cm;
- Behandeln der wässrigen Nanopartikel-Suspension mittels eines Ultraschall-Homogenisators.

19. Verfahren nach Anspruch 18, **dadurch gekennzeichnet, dass** das Behandlungsverfahren einen Zentrifugationsschritt aufweist, bei welchem Partikel mit einer Größe > 250 nm, vorzugsweise mit einer Größe von > 100 nm, aus der Nanopartikel-Suspension abgetrennt werden.

20. Verfahren zur Herstellung einer wässrigen Suspension von Magnetit-Nanopartikeln, **dadurch gekennzeichnet, dass** es folgende Schritte aufweist:
- Suspendieren von magnetisierbaren Nanopartikeln in vollentsalztem Wasser mit einem Leitwert von weniger als 100 µS/cm, vorzugsweise weniger als 5 µS/cm;
- Behandeln der wässrigen Nanopartikel-Suspension mittels Ultraschall;
- Zentrifugieren der wässrigen Nanopartikel-Suspension bei 1000 bis 3000 x g.

21. Sphärische, magnetisierbare Polyvinylalkohol-Mikropartikel, welche durch ein Verfahren gemäß einem der Ansprüche 1 bis 19 hergestellt oder erhältlich sind, und eine Partikelgrößenverteilung im Bereich von 0,5 bis 3 µm, vorzugsweise von 0,5 bis 1 µm, aufweisen und nanopartikuläres magnetisierbares Material, insbesondere Magnetit, in einem Anteil von mindestens 50 Gew.-% enthalten.

22. Sphärische, magnetisierbare Polyvinylalkohol-Mikropartikel nach Anspruch 21, mit einer Partikelgrößenverteilung im Bereich von 1,25 bis 2,25 µm.

23. Sphärische, magnetisierbare Polyvinylalkohol-Mikropartikel, insbesondere Polyvinylalkohol-Mikropartikel nach Anspruch 21 oder 22, **dadurch gekennzeichnet, dass** das Zetapotential der Mikropartikel ≤ -35 mV, besonders bevorzugt ≤ -40 mV beträgt.

24. Sphärische, magnetisierbare Polyvinylalkohol-Mikropartikel, insbesondere Polyvinylalkohol-Mikropartikel nach einem der Ansprüche 21 bis 23, **dadurch gekennzeichnet, dass** die Mikropartikel beim Einsatz in der Real Time Quantitative PCR keine Veränderung des Ct-Wertes hervorrufen oder allenfalls eine Erhöhung des Ct-Wertes um maximal 2 %, vorzugsweise um maximal 1 %, besonders bevorzugt um maximal 0,5 %, bewirken.

25. Sphärische, magnetisierbare Polyvinylalkohol-Mikropartikel nach einem der Ansprüche 21 bis 24, **dadurch gekennzeichnet, dass** die Mikropartikel nanopartikuläres magnetisierbares Material, insbesondere Magnetit, in einem Anteil von mindestens 60 Gew.-%, vorzugsweise mindestens 75 Gew.-%, und insbesondere bis zu 90 Gew.-%, enthalten.

26. Sphärische, magnetisierbare Polyvinylalkohol-Mikropartikel nach einem der Ansprüche 21 bis 25, **dadurch gekennzeichnet, dass** sie funktionelle Gruppen aufweisen, die eine Kopplung von Biomolekülen oder Liganden über kovalente oder nichtkovalente Bindungen ermöglichen.

27. Sphärische, magnetisierbare Polyvinylalkohol-Mikropartikel gemäß einem der Ansprüche 21 bis 26, **dadurch gekennzeichnet, dass** ein oder mehrere Biomoleküle oder Liganden kovalent oder nicht-kovalent an die Partikel gebunden sind, wobei die Biomoleküle oder Liganden vorzugsweise aus der Antikörper, Avidin, Biotin, Protein A, Protein G, Lektine, Oligosaccharide, Oligonucleotide, Enzyme, Enzyminhibitoren, Enzymsubstrate, Rezeptorproteine, Albumin, Gelatine, Glutathion, Aminosäuren, Peptide, Hormone und Neurotransmitter umfassenden Gruppe ausgewählt sind.

28. Verwendung von magnetisierbaren Polyvinylalkohol-Mikropartikeln, die durch ein Verfahren gemäß einem der Ansprüche 1 bis 19 hergestellt wurden, oder von magnetisierbaren Polyvinylalkohol-Mikropartikeln nach einem der Ansprüche 21 bis 26, zur Isolierung, Reinigung oder Anreicherung von Zellen, Nukleinsäuren, Peptiden, Proteinen, Toxinen, Viren, Bakterien, Antikörpern, Enzymen, Antigenen oder Rezeptoren, sowie in diagnostischen oder forensischen Nachweisverfahren, in Immunoassays, bei der DNA-Sequenzierung, PCR-Produkt-Reinigung, Zellfraktionierung, Protein-Isolierung, Affinitätsreinigung, Immunpräzipitation, Gewebetypisierung, Oligonukleotidsynthese oder Peptidsynthese.

## Claims

1. Method for the production of spherical, magnetisable polyvinyl alcohol microparticles having a particle size distribution in the range of 0.5 to 3 *µ*m, comprising the following steps:
- dispersing a nanoparticulate, magnetisable material, preferably magnetite, in an aqueous phase which contains polyvinyl alcohol in dissolved form;
- adding the aqueous phase to an organic phase which is immiscible with said aqueous phase and contains at least one emulsifier, and producing an emulsion by stirring at a temperature of 40 °C or higher;
- adding at least one water-soluble crosslinking agent suitable for crosslinking polyvinyl alcohol, while stirring is continued.

2. Method according to claim 1, **characterised in that** the nanoparticulate magnetisable material comprises magnetisable nanoparticles of a particle size in the range of 5 to 250 nm, preferably 5 to 100 nm, more preferably 5 to 50 nm.

3. Method according to claim 1 or 2, **characterised in that** nanocrystalline magnetite is used as the said nanoparticulate magnetisable material.

4. Method according to any one of the preceding claims, **characterised in that** prior to adding the aqueous phase to the organic phase, said aqueous phase undergoes a treatment by which particles of a size of >250 nm, preferably of a size of >100 nm, are removed, preferably by centrifuging.

5. Method according to any one of the preceding claims, **characterised in that** the concentration of polyvinyl alcohol in the aqueous phase does not exceed 2.0 percent by weight, and preferably amounts to 0.1 to 2.0 percent by weight, more preferably 0.5 to 1.5 percent by weight.

6. Method according to any one of the preceding claims, **characterised in that** the concentration of the nanoparticulate magnetisable material in the aqueous phase is 0.5 to 7.5 percent by weight, preferably 1 to 5 percent by weight.

7. Method according to any one of the preceding claims, **characterised in that** the organic, water-immiscible phase comprises one or more substance(s) selected from the group comprising vegetable oils, synthetic oils, mineral oils, silicone oils and paraffin oils.

8. Method according to any one of the preceding claims, **characterised in that** the proportion of emulsifier contained in the organic phase is 0.1 to 10 percent by weight, preferably 1 to 7.5 percent by weight.

9. Method according to any one of the preceding claims, **characterised in that** the crosslinking agent is selected from the group comprising bifunctional aldehydes, especially glutaraldehyde, as well as acid chlorides and divinyl sulfone.

10. Method according to any one of the preceding claims, **characterised in that** the crosslinking agent is added in liquid form, with the proportion of said crosslinking agent amounting to 0.1 to 10 percent by volume, preferably 1 to 7.5 percent by volume, in each case relative to the aqueous phase.

11. Method according to any one of the preceding claims, **characterised in that** bifunctional aldehydes are used as the crosslinking agent(s) and that the crosslinking reaction is performed with addition of an acid, with 1N to 3N hydrochloric acid at a volume percent of up to 10%, preferably up to 5%, more preferably not exceeding 3.2%, relative to the aqueous phase, being used preferably.

12. Method according to any one of the preceding claims, **characterised in that** the emulsion is produced by stirring at a temperature of at least 50 °C, more preferably at 55 to 65 °C.

13. Method according to any one of the preceding claims, **characterised in that** the emulsion is produced by using a dispersion mixer which operates according to the rotor-stator principle.

14. Method according to one or more of the preceding claims, **characterised by** the following steps:
- dispersing a nanoparticulate, magnetisable material, preferably magnetite, in an aqueous phase which contains polyvinyl alcohol in dissolved form, with the concentration of polyvinyl alcohol in the aqueous phase not exceeding 2.0 percent by weight, and preferably amounting to 0.1 to 2.0 percent by weight, more preferably 0.5 to 1.5 percent by weight;
- adding the aqueous phase to an organic phase which is immiscible with said aqueous phase and contains at least one emulsifier, and producing an emulsion by stirring at a temperature of at least 40 °C, preferably 55 to 65 °C, more preferably 60 °C;
- adding at least one water-soluble crosslinking agent suitable for crosslinking polyvinyl alcohol, while stirring is continued.

15. Method according to one or more of the preceding claims, **characterised by** the following steps:
- dispersing a nanoparticulate, magnetisable material, preferably magnetite, of a particle size not exceeding 250 nm, preferably not exceeding 100 nm, in an aqueous phase which contains polyvinyl alcohol in dissolved form, with the concentration of polyvinyl alcohol in the aqueous phase not exceeding 2.0 percent by weight, and preferably amounting to 0.1 to 2.0 percent by weight, more preferably 0.5 to 1.5 percent by weight;
- adding the aqueous phase to an organic phase which is immiscible with said aqueous phase and contains at least one emulsifier, and producing an emulsion by stirring at a temperature of at least 40 °C, preferably 50 to 65 °C, more preferably 60 °C;
- adding at least one bifunctional aldehyde as crosslinking agent, with addition of 1N to 3N hydrochloric acid at a volume percent of up to 10%, preferably up to 5%, more preferably 3.2%, in each case relative to the aqueous phase.

16. Method according to one or more of the preceding claims, **characterised by** the following steps:
- dispersing a nanoparticulate, magnetisable material, preferably magnetite, in an aqueous phase which contains polyvinyl alcohol in dissolved form, with the concentration of polyvinyl alcohol in the aqueous phase not exceeding 2.0 percent by weight, and preferably amounting to 0.1 to 2.0 percent by weight, more preferably 0.5 to 1.5 percent by weight;
- adding the aqueous phase to an organic phase which is immiscible with said aqueous phase and contains at least one emulsifier, and producing an emulsion by stirring at a temperature of 40 °C or higher, said emulsion being produced using a dispersion mixer which operates according to the rotor-stator principle, at a rotor speed in the range of 500 to 4000 revolutions/min, and the volume of the emulsion being 10 1 or more;
- adding at least one water-soluble crosslinking agent which is suitable for crosslinking polyvinyl alcohol, while stirring is continued.

17. Method according to one or more of the preceding claims, **characterised by** the following steps:
- dispersing a nanoparticulate, magnetisable material, preferably magnetite, in an aqueous phase which contains polyvinyl alcohol in dissolved form, with the concentration of polyvinyl alcohol in the aqueous phase not exceeding 2.0 percent by weight, and preferably amounting to 0.1 to 2.0 percent by weight, more preferably 0.5 to 1.5 percent by weight;
- adding the aqueous phase to an organic phase which is immiscible with said aqueous phase and contains at least one emulsifier, and producing an emulsion by stirring at a temperature of at least 40 °C, preferably 55 to 65 °C, more preferably 60 °C, said emulsion being produced by using a dispersion mixer which operates according to the rotor-stator principle, at a rotor speed in the range of 500 to 4000 revolutions/min, and the volume of the emulsion being 10 1 or more;
- adding at least one bifunctional aldehyde, preferably glutaraldehyde, as crosslinking agent, with addition of 1N to 3N hydrochloric acid at a volume percent of up to 10%, preferably up to 5%, more preferably not exceeding 3.2%, in each case relative to the aqueous phase.

18. Method according to any one of the preceding claims, **characterised in that** the nanoparticulate magnetisable material used, preferably magnetite, is subjected to a treatment method comprising the following steps:
- suspending magnetisable nanoparticles in deionised water having a conductance of less than 100 *µ*S/cm; preferably less than 5 *µ*S/cm;
- treating the aqueous nanoparticle suspension by means of an ultrasound homogeniser.

19. The method according to claim 18, **characterised in that** the treatment method comprises a centrifugation step in which particles of a size of >250 nm, preferably of a size of >100 nm, are separated from the nanoparticle suspension.

20. Method for the production of an aqueous suspension of magnetite nanoparticles, **characterized in that** it comprises the following steps:
- suspending magnetisable nanoparticles in deionised water having a conductance of less than 100 *µ*S/cm, preferably less than 5 *µ*S/cm;
- treating the aqueous nanoparticle suspension with ultrasound;
- centrifuging the aqueous nanoparticle suspension at 1000 to 3000 x g.

21. Spherical, magnetisable polyvinyl alcohol microparticles that can be produced or obtained by a method according to any one of claims 1 to 19 and that have a particle size distribution in the range of 0.5 to 3 *µ*m, preferably 0.5 to 1 *µ*m, and contain nanoparticulate magnetisable material, especially magnetite, at a percentage of at least 50 percent by weight.

22. Spherical, magnetisable polyvinyl alcohol microparticles according to claim 21, having a particle size distribution in the range of 1.25 to 2.25 *µ*m.

23. Spherical, magnetisable polyvinyl alcohol microparticles, particularly polyvinyl alcohol microparticles according to claim 21 or 22, **characterised in that** the zeta potential of the microparticles is ≤-35 mV, more preferably ≤-40 mV.

24. Spherical, magnetisable polyvinyl alcohol microparticles, particularly polyvinyl alcohol microparticles according to any one of claims 21 to 23, **characterised in that** when used in real time quantitative PCR the micro-particles do not cause a change in the Ct value or, at the most, cause an increase of the Ct value by at most 2%, preferably by at most 1%, more preferably by at most 0.5%.

25. Spherical, magnetisable polyvinyl alcohol microparticles according to any one of claims 21 to 24, **characterised in that** the microparticles contain nanoparticulate magnetisable material, especially magnetite, at a percentage of at least 60 percent by weight, preferably at least 75 percent by weight, and more particularly up to 90 percent by weight.

26. Spherical, magnetisable polyvinyl alcohol microparticles according to any one of claims 21 to 25, **characterised in that** they comprise functional groups which enable the coupling of biomolecules or ligands via covalent or non-covalent bonds.

27. Spherical, magnetisable polyvinyl alcohol microparticles according to any one of claims 21 to 26, **characterised in that** one or more biomolecules or ligands are covalently or non-covalently bound to the particles, said biomolecules or ligands preferably being selected from the group comprising antibodies, avidin, biotin, protein A, protein G, lectins, oligosaccharides, oligonucleotides, enzymes, enzyme inhibitors, enzyme substrates, receptor proteins, albumin, gelatine, glutathione, amino acids, peptides, hormones and neurotransmitters.

28. The use of magnetisable polyvinyl alcohol microparticles which have been prepared by a method according to any one of claims 1 to 19, or of magnetisable polyvinyl alcohol microparticles according to any one of claims 21 to 26, for isolation, purification or enrichment of cells, nucleic acids, peptides, proteins, toxins, viruses, bacteria, antibodies, enzymes, antigens or receptors, as well as in diagnostic or forensic detection methods, in immunoassays, in DNA sequencing, PCR product purification, cell fractionation, protein isolation, affinity purification, immuno-precipitation, tissue typing, oligo-nucleotide synthesis or peptide synthesis.

## Revendications

1. Procédé pour la fabrication de microparticules sphériques, magnétisables de poly(alcool vinylique) présentant une répartition des grosseurs de particule dans la plage de 0,5 à 3 µm, comprenant les étapes suivantes:
- disperser un matériau nanoparticulaire, magnétisable, de préférence de la magnétite, dans une phase aqueuse qui contient du poly(alcool vinylique) sous forme dissoute ;
- ajouter la phase aqueuse à une phase organique, qui n'est pas miscible à la phase aqueuse et qui contient au moins un émulsifiant, et générer une émulsion par agitation à une température de 40°C ou plus ;
- ajouter au moins une substance de réticulant, soluble dans l'eau, appropriée pour la réticulation de poly(alcool vinylique) tout en continuant à agiter.

2. Procédé selon la revendication 1, **caractérisé en ce que** le matériau nanoparticulaire, magnétisable est constitué par des nanoparticules magnétisables présentant une grosseur de particule dans la plage de 5 à 250 nm, de préférence de 5 à 100 nm, de manière particulièrement préférée de 5 à 50 nm.

3. Procédé selon la revendication 1 ou 2, **caractérisé en ce qu'**on utilise, comme matériau nanoparticulaire, magnétisable, de la magnétite nanocristalline.

4. Procédé selon l'une quelconque des revendications précédentes, **caractérisé en ce que** la phase aqueuse, avant l'addition à la phase organique, est soumise à un traitement par lequel des particules présentant une grosseur > 250 nm, en particulier une grosseur > 100 nm, sont éliminées, de préférence par centrifugation.

5. Procédé selon l'une quelconque des revendications précédentes, **caractérisé en ce que** la concentration en poly(alcool vinylique) dans la phase aqueuse est d'au maximum 2,0% en poids, de préférence de 0,1 à 2,0% en poids, de manière particulièrement préférée de 0,5 à 1,5% en poids.

6. Procédé selon l'une quelconque des revendications précédentes, **caractérisé en ce que** la concentration en matériau nanoparticulaire, magnétisable dans la phase aqueuse est de 0,5 à 7,5% en poids, de préférence de 1 à 5% en poids.

7. Procédé selon l'une quelconque des revendications précédentes, **caractérisé en ce que** la phase organique, non miscible à l'eau, comprend une ou plusieurs substances choisies dans le groupe comprenant les huiles végétales, les huiles synthétiques, les huiles minérales, les huiles de silicone et les huiles de paraffine.

8. Procédé selon l'une quelconque des revendications précédentes, **caractérisé en ce que** la proportion d'émulsifiant dans la phase organique est de 0,1 à 10% en poids, de préférence de 1 à 7,5% en poids.

9. Procédé selon l'une quelconque des revendications précédentes, **caractérisé en ce que** la substance de réticulant est choisie dans le groupe comprenant les aldéhydes difonctionnels, en particulier le glutaraldéhyde, ainsi que les chlorures d'acide et la divinylsulfone.

10. Procédé selon l'une quelconque des revendications précédentes, **caractérisé en ce que** la substance de réticulant est ajoutée sous forme liquide, la proportion étant de 0,1 à 10% en volume, de préférence de 1 à 7,5% en volume, à chaque fois par rapport à la phase aqueuse.

11. Procédé selon l'une quelconque des revendications précédentes, **caractérisé en ce qu'**on utilise, comme substance(s) de réticulant, des aldéhydes difonctionnels et la réaction de réticulation est réalisée avec addition d'un acide, de l'acide chlorhydrique de 1 N à 3 N en une proportion volumique de jusqu'à 10%, de préférence de jusqu'à 5%, en particulier d'au plus 3,2%, par rapport à la phase aqueuse, étant de préférence utilisé.

12. Procédé selon l'une quelconque des revendications précédentes, **caractérisé en ce que** la génération de l'émulsion est réalisée par agitation à une température d'au moins 50°C, de manière particulièrement préférée à 55 jusqu'à 65°C.

13. Procédé selon l'une quelconque des revendications précédentes, **caractérisé en ce que** la préparation de l'émulsion a lieu au moyen d'un agitateur de dispersion fonctionnant selon le principe de rotor-stator.

14. Procédé selon l'une ou plusieurs des revendications précédentes, **caractérisé par** les étapes suivantes:
- disperser un matériau nanoparticulaire, magnétisable, de préférence de la magnétite, dans une phase aqueuse qui contient du poly(alcool vinylique) sous forme dissoute, la concentration en poly(alcool vinylique) dans la phase aqueuse étant d'au maximum 2,0% en poids, de préférence de 0,1 à 2,0% en poids, de manière particulièrement préférée de 0,5 à 1,5% en poids ;
- ajouter la phase aqueuse à une phase organique qui n'est pas miscible à la phase aqueuse et qui contient au moins un émulsifiant et générer une émulsion par agitation à une température d'au moins 40°C, de préférence à 55 jusqu'à 65 °C, en particulier à 60°C ;
- ajouter au moins une substance de réticulation, soluble dans l'eau, appropriée pour la réticulation de poly(alcool vinylique) tout en continuant à agiter.

15. Procédé selon l'une ou plusieurs des revendications précédentes, **caractérisé par** les étapes suivantes:
- disperser un matériau nanoparticulaire, magnétisable, de préférence de la magnétite, présentant une grosseur de particule d'au plus 250 nm, de préférence d'au plus 100 nm, dans une phase aqueuse qui contient du poly(alcool vinylique) sous forme dissoute, la concentration en poly(alcool vinylique) dans la phase aqueuse étant d'au maximum 2,0% en poids, de préférence de 0,1 à 2,0% en poids, de manière particulièrement préférée de 0,5 à 1,5% en poids ;
- ajouter la phase aqueuse à une phase organique, qui n'est pas miscible à la phase aqueuse et qui contient au moins un émulsifiant, et générer une émulsion par agitation à une température d'au moins 40°C, de préférence à 50 jusqu'à 65°C, en particulier à 60°C ;
- ajouter au moins un aldéhyde difonctionnel comme réticulant, avec addition d'acide chlorhydrique de 1 N à 3 N en une proportion volumique de jusqu'à 10%, de préférence de jusqu'à 5%, en particulier d'au plus 3,2%, par rapport à la phase aqueuse.

16. Procédé selon l'une ou plusieurs des revendications précédentes, **caractérisé par** les étapes suivantes:
- disperser un matériau nanoparticulaire, magnétisable, de préférence de la magnétite, dans une phase aqueuse qui contient du poly(alcool vinylique) sous forme dissoute, la concentration en poly(alcool vinylique) dans la phase aqueuse étant d'au maximum 2,0% en poids, de préférence de 0,1 à 2,0% en poids, de manière particulièrement préférée de 0,5 à 1,5% en poids ;
- ajouter la phase aqueuse à une phase organique, qui n'est pas miscible à la phase aqueuse et qui contient au moins un émulsifiant, et générer une émulsion par agitation à une température de 40°C ou plus, la préparation de l'émulsion ayant lieu au moyen d'un agitateur de dispersion fonctionnant selon le principe de rotor-stator à une vitesse de rotation du rotor dans la plage de 500 à 4000 tours/minute et le volume de l'émulsion étant de 10 l ou plus ;
- ajouter au moins une substance de réticulation, soluble dans l'eau, appropriée pour la réticulation de poly(alcool vinylique) tout en continuant à agiter.

17. Procédé selon l'une ou plusieurs des revendications précédentes, **caractérisé par** les étapes suivantes:
- disperser un matériau nanoparticulaire, magnétisable, de préférence de la magnétite, dans une phase aqueuse qui contient du poly(alcool vinylique) sous forme dissoute, la concentration en poly(alcool vinylique) dans la phase aqueuse étant d'au maximum 2,0% en poids, de préférence de 0,1 à 2,0% en poids, de manière particulièrement préférée de 0,5 à 1,5% en poids ;
- ajouter la phase aqueuse à une phase organique, qui n'est pas miscible à la phase aqueuse et qui contient au moins un émulsifiant, et générer une émulsion par agitation à une température d'au moins 40°C, de préférence à 55 jusqu'à 65°C, en particulier à 60°C, la préparation de l'émulsion ayant lieu au moyen d'un agitateur de dispersion fonctionnant selon le principe de rotor-stator à une vitesse de rotation du rotor dans la plage de 500 à 4000 tours/minute et le volume de l'émulsion étant de 10 l ou plus ;
- ajouter au moins un aldéhyde difonctionnel, de préférence du glutaraldéhyde, comme réticulant, avec addition d'acide chlorhydrique de 1 N à 3 N en une proportion volumique de jusqu'à 10%, de préférence de jusqu'à 5%, en particulier d'au plus 3,2%, par rapport à la phase aqueuse.

18. Procédé selon l'une quelconque des revendications précédentes, **caractérisé en ce que** le matériau nanoparticulaire, magnétisable, utilisé, de préférence la magnétite, est soumis à un procédé de traitement qui présente les étapes suivantes:
- mettre en suspension des nanoparticules magnétisables dans de l'eau déminéralisée présentant une conductivité inférieure à 100 µS/cm, de préférence inférieure à 5 µS/cm ;
- traiter la suspension aqueuse de nanoparticules au moyen d'un homogénéisateur à ultrasons.

19. Procédé selon la revendication 18, **caractérisé en ce que** le procédé de traitement présente une étape de centrifugation, lors de laquelle des particules présentant une grosseur > 250 nm, de préférence une grosseur > 100 nm, sont séparées de la suspension de nanoparticules.

20. Procédé pour la préparation d'une suspension aqueuse de nanoparticules de magnétite, **caractérisé en ce qu'**il présente les étapes suivantes:
- mettre en suspension des nanoparticules magnétisables dans de l'eau déminéralisée présentant une conductivité inférieure à 100 µS/cm, de préférence inférieure à 5 µS/cm ;
- traiter la suspension aqueuse de nanoparticules par des ultrasons ;
- centrifuger la suspension aqueuse de nanoparticules à 1000 jusqu'à 3000 x g.

21. Microparticules sphériques, magnétisables de poly(alcool vinylique), qui sont préparées ou qui peuvent être obtenues par un procédé selon l'une quelconque des revendications 1 à 19 et qui présentent une répartition des grosseurs de particule dans la plage de 0,5 à 3 µm, de préférence de 0,5 à 1 µm et qui contiennent un matériau nanoparticulaire, magnétisable, en particulier de la magnétite, en une proportion d'au moins 50 % en poids.

22. Microparticules sphériques, magnétisables de poly(alcool vinylique) selon la revendication 21, présentant une répartition de grosseurs de particule dans la plage de 1,25 à 2,25 µm.

23. Microparticules sphériques, magnétisables de poly(alcool vinylique), en particulier microparticules de poly(alcool vinylique) selon la revendication 21 ou 22, **caractérisées en ce que** le potentiel zêta des microparticules est ≤ -35 mV, de manière particulièrement préférée ≤ -40 mV.

24. Microparticules sphériques, magnétisables de poly(alcool vinylique), en particulier microparticules de poly(alcool vinylique) selon l'une quelconque des revendications 21 à 23, **caractérisées en ce que** les microparticules, lors de l'utilisation dans une PCR quantitative en temps réel, ne provoquent pas de modification de la valeur Ct ou provoquent éventuellement une augmentation de la valeur Ct d'au maximum 2%, de préférence d'au maximum 1%, de manière particulièrement préférée d'au maximum 0,5%.

25. Microparticules sphériques, magnétisables de poly(alcool vinylique) selon l'une quelconque des revendications 21 à 24, **caractérisées en ce que** les microparticules contiennent un matériau nanoparticulaire, magnétisable, en particulier de la magnétite, en une proportion d'au moins 60% en poids, de préférence d'au moins 75% en poids et en particulier de jusqu'à 90% en poids.

26. Microparticules sphériques, magnétisables de poly(alcool vinylique) selon l'une quelconque des revendications 21 à 25, **caractérisées en ce qu'**elles présentent des groupes fonctionnels qui permettent un couplage de biomolécules ou de ligands via des liaisons covalentes ou non covalentes.

27. Microparticules sphériques, magnétisables de poly(alcool vinylique) selon l'une quelconque des revendications 21 à 26, **caractérisées en ce qu'**une ou plusieurs biomolécules ou un ou plusieurs ligands sont liés par covalence ou de manière non covalente aux particules, les biomolécules ou les ligands étant de préférence choisi(e)s dans le groupe comprenant les anticorps, l'avidine, la biotine, la protéine A, la protéine G, les lectines, les oligosaccharides, les oligonucléotides, les enzymes, les inhibiteurs d'enzymes, les substrats d'enzymes, les protéines réceptrices, l'albumine, la gélatine, le glutathion, les acides aminés, les peptides, les hormones et les neurotransmetteurs.

28. Utilisation de microparticules magnétisables de poly(alcool vinylique), qui ont été préparées par un procédé selon l'une quelconque des revendications 1 à 19, ou de microparticules magnétisables de poly(alcool vinylique) selon l'une quelconque des revendications 21 à 26 pour isoler, purifier ou enrichir des cellules, des acides nucléiques, des peptides, des protéines, des toxines, des virus, des bactéries, des anticorps, des enzymes, des antigènes ou des récepteurs ainsi que dans des procédés de détection diagnostiques ou médico-légaux, dans des dosages immunologiques, lors du séquençage de l'ADN, lors de la purification de produits par PCR, lors du fractionnement cellulaire, lors de l'isolement de protéines, lors d'une purification par affinité, lors d'une immunoprécipitation, lors d'un typage tissulaire, lors de la synthèse d'oligonucléotides ou de la synthèse de peptides.
